# EUROPEAN PATENT APPLICATION

(11) **EP 1 340 755 A1**
(43) Date of publication of application: **03.09.2003**
(21) Application number: 01982772.4
(22) Date of filing: 13.11.2001
(51) Int. Cl.: C07D 471/06, A61K 31/4375, A61P 31/04, A61P 1/04

(54) **ANTI-HELICOBACTERIAL AGENTS**

(30) Priority: 14.11.2000 JP 2000347348
(71) Applicant: SHIONOGI & CO., LTD., Osaka-shi, Osaka 541-0045 (JP)
(72) Inventor: SUGIMORI, Giichi, c/o SHIONOGI & CO., LTD., Koka-gun, Shiga 520-3423 (JP); OHTSUKA, Toshikazu, c/o SHIONOGI & CO., LTD., Koka-gun, Shiga 520-3423 (JP); MASUI, Moriyasu, c/o SHIONOGI & CO., LTD., Koka-gun, Shiga 520-3423 (JP)
(74) Representative: Tanner, James Percival
(86) International application number: JP0109929
(87) International publication number: WO02040479

(57) **Abstract**

The object of the present invention is to provide a compound having an anti-Helicobacter action. The present invention provides a pharmaceutical composition containing a compound represented by the following formula: 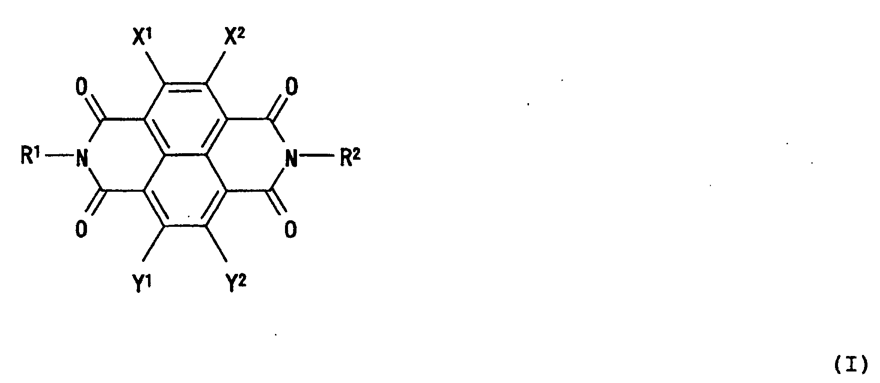 or a pharmaceutically acceptable salt or hydrate thereof. The present invention alone is useful as an anti-Helicobacter agent.

## Description

### TECHNICAL FIELD

The present invention relates to a compound having anti-Helicobacter activity and an anti-Helicobacter pharmaceutical composition containing the same. The pharmaceutical composition of the present invention is useful, for example, as an antiulcer agent, and an anti-inflammatory agent for the digestive organs.

### BACKGROUND ART

As therapeutic agents for ulcers, such as, for example, gastric and duodenal ulcers, various types of pharmaceutical agents, such as, for example, anti-gastrin agents, histamine H₂ receptor antagonists, and proton pump inhibitors, have been developed. Unfortunately, all of such pharmaceutical agents have a high chance for recurrence of the ulcers after discontinuation of therapy with the drug.

Helicobacter pylori is a bacterium of the genus Helicobacter, which has been identified as a bacterium capable of living within the stomach, which is strongly acidic. This bacterium is a Gram-negative Spirillaceae of the genus Helicobacter, and has been confirmed to be one of the causes of human gastric and duodenal ulcers, etc. (Campylobacter pylori and peptic ulcer disease. Gastroenterology 96:615-625(1989)). Helicobacter pylori has also been designated as a risk factor for gastric cancer by the World Health Organization (WHO) since 1994.

It is suggested that in clinical tests, when Gram-negative Spirillaceae of the genus Helicobacter is eradicated from tissues of the upper digestive organs, ulcers disappear and further the chances for recurrence of the ulcers are reduced (Graham DY et al., Ann. Intern. Med. 116:705-708 (1992) ; Marshall BJ et al., Lancet 2:1437-1442 (1988) ; Kihira, K. et al., Rinsyo-to-Biseibutu [Clinics and Microorganisms], Vol. 24, No. 3, 335-339 (1997); Satoh, K. et al., Recent Findings of Helicobacter pylori (edited by The Japanese Society of Gastroenterology, Nakayama Syoten), 265-273; Takimoto,T et al., Recent Findings of Helicobacter pylori (edited by The Japanese Society of Gastroenterology, Nakayama Syoten), 287-293). Under such circumstances, the use of antibacterial agents has begun in the treatment of peptic ulcers. To date, their range of application has extended to encompasses not only peptic ulcer but also chronic gastritis, early gastric cancer (e.g., post-excision use in treatment of gastric cancer), and diseases or disorders of digestive organs including dyspepsia (Kihira, K. et al., Rinsyo-to-Biseibutu [Clinics and Microorganisms], Vol. 24, No. 3, 335-339 (1997)).

However, when the compounds which have been used to date (including existing antibacterial agents) areusedalone, their effects are insufficient or absent, so that a sufficient bacterial eradication effect is not obtained. Actually, H. pylori is sensitive, in in vitro tests, to antibiotics such as, for example, penicillin, cephalosporin, macrolide, and nitroimidazole. However, when these antibiotics are used alone in in vivo tests, a bacterial eradication effect is not obtained. According to Chiba et al.'s report on the effects of a mixture of agents on bacterial eradication, the average eradication rate was 18.6% for a single agent, 48.5% for a mixture of two agents, and 82% for a mixture of three agents (Chiba N. et al., Am J Gastoenterol 87:1716-1727 (1992)). The reasons why a single agent cannot cause a sufficient bacterial eradication effect include that the antibacterial activity of an orally administered antibiotic is attenuated and killed by gastric acid; a delivered antibiotic does not have an effective concentration to bacteria present within a mucous layer; and H. pylori becomes resistant to pharmaceutical agents, for example (Axon, AT, Scand J Gastroenterl 29:16-23 (1994)).

For those reasons, the bacterial eradication treatment of peptic ulcers employing a single antibiotic is not currently conducted. For example, a triple-drug therapy employing a bismuth preparation and two antibiotics (e.g., metronidazole/amoxicillin and metronidazole/tetracycline) is conducted (Am. J. Gastroenterol. (1992)). According to a guideline for eradication of Helicobacter pylori, a combination of a proton pump inhibitor (e.g., omeprazole) and an antibiotic (e.g., amoxicillin) is advocated as a first choice (NIH consensus development conference. 1994. Helicobacter pylori in peptic ulcer diseases. JAMA 272:65-69).

### (Problems to be Solved by the Invention)

Unfortunately, the above-described multiple-drug therapy causes side effects such as, for example, nausea and diarrhea, or is likely to have a significant reduction in compliance. There is, therefore, a demand for development of a novel anti-Helicobacter agent which is well effective when used alone. There are a number of problems with novel antibiotics obtained by improving a conventional antibiotic in overcoming resistance. For this reason, there is a need for development of a totally new generation antibacterial agent.

### DISCLOSURE OF THE INVENTION

In order to achieve the above-described objects, the present invention provides the following:

The present invention provides a compound represented by the following formula, or a pharmaceutically acceptable salt or hydrate thereof, thereby solving the above-described problems:

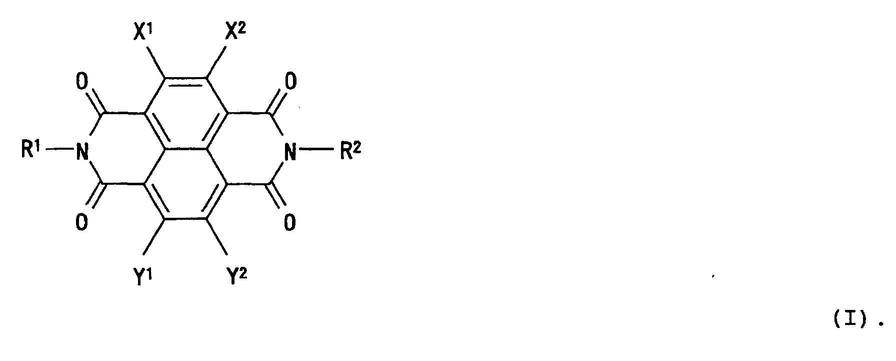

In this case, R¹ and R² are separately selected from the group consisting of hydrogen, alkyl, substituted alkyl, cycloalkyl, substituted cycloalkyl, alkenyl, substituted alkenyl, cycloalkenyl, substituted cycloalkenyl, alkynyl, substituted alkynyl, alkoxy, substituted alkoxy, carbocyclic group, substituted carbocyclic group, heterocyclic group, substituted heterocyclic group, halogen, hydroxy, substituted hydroxy, thiol, substituted thiol, cyano, nitro, amino, substitutedamino, carboxy, substituted carboxy, acyl, substituted acyl, thiocarboxy, substituted thiocarboxy, amide, substituted amide, substituted carbonyl, substituted thiocarbonyl, substituted sulfonyl, and substituted sulfinyl.

X¹, X², Y¹, and Y² are separately selected from the group consisting of hydrogen, halogen, alkoxy, substituted alkoxy, amino, substituted amino, alkylthio, substituted alkylthio, arylthio, substituted arylthio, nitro, carboxy, substituted carboxy, acyl, substituted acyl, and substituted sulfonyl.

In one preferred embodiment, R¹ and R² may be separately heterocyclic group, substituted heterocyclic group, alkyl, or substituted alkyl; and X¹, X², Y¹ and Y² may be all hydrogen.

In one more preferred embodiment, R¹ and R² may be separately heterocyclic group, substituted heterocyclic group, alkyl, or substituted alkyl; and X¹, X², Y¹ and Y² may be separately substituents selected from the group consisting of alkyl, halogen, and hydrogen.

In one more preferred embodiment, R¹ and R² may be separately substituted alkyl; the substituent of the substituted alkyl may be pyridyl, hydroxy, substituted carboxy, alkoxy, or substituted amino; and X¹, X², Y¹ and Y² may be all hydrogen.

In one more preferred embodiment, R¹ and R² may be separately heterocyclic group, substituted heterocyclic group, alkyl, or substituted alkyl; and X¹, X², Y¹ and Y² may be all hydrogen.

In one more preferred embodiment, R¹ and R² may be separately selected from the group consisting of pyridyl, substituted pyridyl, pyrimidyl, substituted pyrimidyl, pyrazyl, substituted pyrazyl, quinolyl, substituted quinolyl, isoquinolyl, and substituted isoquinolyl.

In one more preferred embodiment, R¹ and R² may be separately substituted alkyl; and the substituent of the substituted alkyl may be pyridyl, hydroxy, substituted carboxy, alkoxy, or substituted amino.

In one embodiment, the present invention provides a pharmaceutical composition containing the above-described compound or a pharmaceutically acceptable salt or hydrate thereof, and a pharmaceutically acceptable carrier.

In another embodiment, the present invention provides an anti-Helicobacter pharmaceutical composition containing the above-described compound or a pharmaceutically acceptable salt or hydrate thereof, and a pharmaceutically acceptable carrier.

The composition of the present invention may further contain at least one drug selected from the group of consisting of an antibacterial agent, a mucosal protection agent promoter, an anti-gastrin agent, a H₂ receptor antagonist, a proton pump inhibitor, a bismuth preparation, and a gastrointestinal drug. The compound or composition of the present invention is used to enhance the efficacy of these drugs.

Various diseases may be treated with the composition of the present invention. The composition of the present invention may be preferably useful in treatment of gastric ulcer, duodenal ulcer, or gastritis, and also other diseases (e.g., other digestive organ diseases such as gastric cancer, dyspepsia, etc.).

The pathogens to be treated with the composition of the present invention include Helicobacter. Specific examples of Helicobacter may include Helicobacter pylori or Helicobacter felis.

In another aspect, the present invention provides a method for treating or preventing a disease caused by Helicobacter, or preventing the recurrence of the disease. The method comprises the step of:
a) administering to a patient with a disease a formulation containing a compound represented by the following formula: 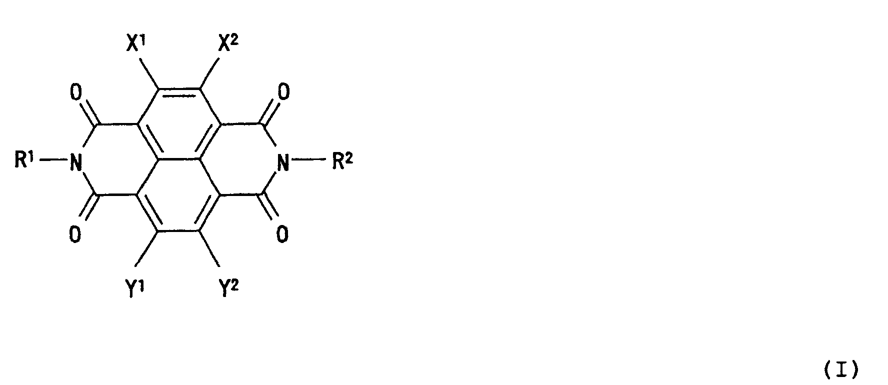
(wherein R¹ and R² are separately selected from the group consisting of hydrogen, alkyl, substituted alkyl, cycloalkyl, substituted cycloalkyl, alkenyl, substituted alkenyl, cycloalkenyl, substituted cycloalkenyl, alkynyl, substituted alkynyl, alkoxy, substituted alkoxy, carbocyclic group, substituted carbocyclic group, heterocyclic group, substituted heterocyclic group, halogen, hydroxy, substituted hydroxy, thiol, substituted thiol, cyano, nitro, amino, substitutedamino, carboxy, substituted carboxy, acyl, substituted acyl, thiocarboxy, substituted thiocarboxy, amide, substituted amide, substituted carbonyl, substituted thiocarbonyl, substituted sulfonyl, and substituted sulfinyl; and
X¹, X², Y¹ and Y² are separately selected from the group consisting of hydrogen, halogen, alkoxy, substituted alkoxy, amino, substituted amino, alkylthio, substituted alkylthio, arylthio, substituted arylthio, nitro, carboxy, substituted carboxy, acyl, substituted acyl, and substituted sulfonyl), or a pharmaceutically acceptable salt or hydrate thereof.

In one preferred embodiment, in the above-described method, R¹ and R² may be separately heterocyclic group, substituted heterocyclic group, alkyl, or substituted alkyl; and X¹, X², Y¹ and Y² may be all hydrogen.

In one more preferred embodiment, R¹ and R² may be separately heterocyclic group, substituted heterocyclic group, alkyl, or substituted alkyl; and X¹, X², Y¹ and Y² may be separately substituents selected from the group consisting of alkyl, halogen, and hydrogen.

In one more preferred embodiment, in the above-described method, R¹ and R² may be separately substituted alkyl; the substituent of the substituted alkyl may be pyridyl, hydroxy, substituted carboxy, alkoxy, or substituted amino; and X¹, X², Y¹ and Y² may be all hydrogen.

In one more preferred embodiment, in the above-described method, R¹ and R² may be separately heterocyclic group, substituted heterocyclic group, alkyl, or substituted alkyl; and X¹, X², Y¹ and Y² may be all hydrogen.

In one more preferred embodiment, R¹ and R² may be selected from the group consisting of pyridyl, substituted pyridyl, pyrimidyl, substituted pyrimidyl, pyrazyl, substituted pyrazyl, quinolyl, substituted quinolyl, isoquinolyl, and substituted isoquinolyl.

In one more preferred embodiment, R¹ and R² may be separately substituted alkyl; and the substituent of the substituted alkyl may be pyridyl, hydroxy, substituted carboxy, alkoxy, or substituted amino.

In another embodiment, the formulation may contain a pharmaceutically acceptable carrier.

In another embodiment, the formulation may further contain at least one drug selected from the group of consisting of an antibacterial agent, a mucosal protection agent promoter, an anti-gastrin agent, a H₂ receptor antagonist, a proton pump inhibitor, a bismuth preparation, and a gastrointestinal drug. More preferably, at least two of these drugs may be contained in the formulation.

In another embodiment, the above-described disease may be gastric ulcer, duodenal ulcer, or gastritis.

In another embodiment, the above-described Helicobacter may be Helicobacter pylori or Helicobacter felis.

In another aspect, the present invention provides the use of the compound of the present invention for use in treating or preventing a disease caused by Helicobacter, or preventing the recurrence of the disease.

### BEST MODE FOR CARRYING OUT THE INVENTION

It should be understood throughout the present specification that articles for a singular form (e.g., "a", "an", "the", etc. in English; "ein", "der", "das", "die", etc. and their inflections in German; "un", "une", "la", "le", etc. in French; articles, adjectives, etc. in other languages) include the concept of their plurality unless otherwise mentioned. It should be also understood that the terms as used herein have definitions typically used in the art unless otherwise mentioned.

The term "alkyl" refers to a monovalent group which is generated by a hydrogen atom being removed from an aliphatic hydrocarbon, such as, for example, methane, ethane, and propane, which is generally represented by CₙH₂ₙ₊₁- (wherein n is a positive integer) . Alkyl may be a straight chain or a branched chain. The term "substituted alkyl" as used herein refers to alkyl in which one or more H are substituted with substituent(s) defined below. Specific examples may be C1-C2 alkyl, C1-C3 alkyl, C1-C4 alkyl, C1-C5 alkyl, C1-C6 alkyl, C1-C7 alkyl, C1-C8 alkyl, C1-C9 alkyl, C1-C10 alkyl, C1-C11 alkyl or C1-C12 alkyl, C1-C2 substituted alkyl, C1-C3 substituted alkyl, C1-C4 substituted alkyl, C1-C5 substituted alkyl, C1-C6 substituted alkyl, C1-C7 substituted alkyl, C1-C8 substituted alkyl, C1-C9 substituted alkyl, C1-C10 substituted alkyl, C1-C11 substituted alkyl or C1-C12 substituted alkyl. In this case, for example, C1-C10 alkyl refers to straight or branched chain alkyl having 1-10 carbon atoms. Examples of C1-C10 alkyl include methyl (CH₃-), ethyl (C₂H₅-), n-propyl (CH₃CH₂CH₂-), isopropyl ((CH₃)₂CH-), n-butyl (CH₃CH₂CH₂CH₂-), n-pentyl (CH₃CH₂CH₂CH₂CH₂-), n-hexyl (CH₃CH₂CH₂CH₂CH₂CH₂-), n-heptyl (CH₃CH₂CH₂CH₂CH₂CH₂CH₂-), n-octyl (CH₃CH₂CH₂CH₂CH₂CH₂CH₂CH₂-), n-nonyl (CH₃CH₂CH₂CH₂CH₂CH₂CH₂CH₂CH₂-), n-decyl (CH₃CH₂CH₂CH₂CH₂CH₂CH₂CH₂CH₂CH₂-), -C (CH₃)₂CH₂CH₂CH(CH₃)₂, and -CH₂CH(CH₃)₂, etc. For example, C1-C10 substituted alkyl is C1-C10 alkyl in which one or more hydrogen atoms are substituted with substituent(s).

The term "cycloalkyl" refers to alkyl having a cyclic structure. The term "substituted cycloalkyl" refers to cycloalkyl in which one or more H are substituted with substituent(s) defined below. Specific examples may be C3-C4 cycloalkyl, C3-C5 cycloalkyl, C3-C6 cycloalkyl, C3-C7 cycloalkyl, C3-C8 cycloalkyl, C3-C9 cycloalkyl, C3-C10 cycloalkyl, C3-C11 cycloalkyl, C3-C12 cycloalkyl, C3-C4 substituted cycloalkyl, C3-C5 substituted cycloalkyl, C3-C6 substituted cycloalkyl, C3-C7 substituted cycloalkyl, C3-C8 substituted cycloalkyl, C3-C9 substituted cycloalkyl, C3-C10 substituted cycloalkyl, C3-C11 substituted cycloalkyl, or C3-C12 substituted cycloalkyl. For example, cycloalkyls include cyclopropyl, cyclohexyl, etc.

The term "alkenyl" refers to a monovalent group which is generated by a hydrogen atom being removed from aliphatic hydrocarbon having one double bond within the molecule, such as, for example, ethylene and propylene, and which is generally represented by CₙH₂ₙ₋₁- (wherein n is a positive integer which is greater than or equal to 2). The term "substituted alkenyl" refers to alkenyl in which one or more H are substituted with substituent(s) defined below. Specific examples may be C2-C3 alkenyl, C2-C4 alkenyl, C2-C5 alkenyl, C2-C6 alkenyl, C2-C7 alkenyl, C2-C8 alkenyl, C2-C9 alkenyl, C2-C10 alkenyl, C2-C11 alkenyl or C2-C12 alkenyl, C2-C3 substituted alkenyl, C2-C4 substituted alkenyl, C2-C5 substituted alkenyl, C2-C6 substituted alkenyl, C2-C7 substituted alkenyl, C2-C8 substituted alkenyl, C2-C9 substituted alkenyl, C2-C10 substituted alkenyl, C2-C11 substituted alkenyl or C2-C12 substituted alkenyl. In this case, for example, C2-C10 alkenyl refers to straight or branched chain alkenyl having 2-10 carbon atoms, including vinyl (CH₂=CH-), allyl (CH₂=CHCH₂-), CH₃CH=CH-, etc. Further, for example, C2-C10 substituted alkenyl refers to C2-C10 alkenyl in which one or more hydrogen atoms are substituted with substituent(s).

The term "cycloalkenyl" refers to alkenyl having a cyclic structure. The term "substituted cycloalkenyl" refers to cycloalkenyl in which one or more H are substituted with substituent(s) defined below. Specific examples may be C3-C4 cycloalkenyl, C3-C5 cycloalkenyl, C3-C6 cycloalkenyl, C3-C7 cycloalkenyl, C3-C8 cycloalkenyl, C3-C9 cycloalkenyl, C3-C10 cycloalkenyl, C3-C11 cycloalkenyl, C3-C12 cycloalkenyl, C3-C4 substituted cycloalkenyl, C3-C5 substituted cycloalkenyl, C3-C6 substituted cycloalkenyl, C3-C7 substituted cycloalkenyl, C3-C8 substituted cycloalkenyl, C3-C9 substituted cycloalkenyl, C3-C10 substituted cycloalkenyl, C3-C11 substituted cycloalkenyl or C3-C12 substituted cycloalkenyl. For example, preferable cycloalkenyls include 1-cyclopentenyl, 2-cyclohexenyl, etc.

The term "alkynyl" refers to a monovalent group which is generated by a hydrogen atom being removed from aliphatic hydrocarbon having a triple bond within the molecule, such as acetylene, and which is generally represented by CₙH₂ₙ₋₃- (where n is a positive integer which is greater than or equal to 2). The term "substituted alkynyl" refers to alkynyl in which one or more H are substituted with substituent(s) defined below. Specific examples may be C2-C3 alkynyl, C2-C4 alkynyl, C2-C5 alkynyl, C2-C6 alkynyl, C2-C7 alkynyl, C2-C8 alkynyl, C2-C9 alkynyl, C2-C10 alkynyl, C2-C11 alkynyl, C2-C12 alkynyl, C2-C3 substituted alkynyl, C2-C4 substituted alkynyl, C2-C5 substituted alkynyl, C2-C6 substituted alkynyl, C2-C7 substituted alkynyl, C2-C8 substituted alkynyl, C2-C9 substituted alkynyl, C2-C10 substituted alkynyl, C2-C11 substituted alkynyl or C2-C12 substituted alkynyl. In this case, for example, C2-C10 alkynyl refers to straight or branched chain alkynyl having 2-10 carbon atoms, including ethynyl (CH≡C-), 1-propynyl (CH₃C≡C-), etc. Further, for example, C2-C10 substituted alkynyl refers to C2-C10 alkynyl in which one or more hydrogen atoms are substituted with substituent(s).

The term "alkoxy" refers to a monovalent group which is generated by a hydrogen atom being removed from the hydroxy group of an alcohol, and which is generally represented by CₙH₂ₙ₊₁O- (where n is an integer which is greater than or equal to 1). The term "substituted alkoxy" refers to alkoxy in which one or more H are substituted with substituent(s) defined below. Specific examples may be C1-C2 alkoxy, C1-C3 alkoxy, C1-C4 alkoxy, C1-C5 alkoxy, C1-C6 alkoxy, C1-C7 alkoxy, C1-C8 alkoxy, C1-C9 alkoxy, C1-C10 alkoxy, C1-C11 alkoxy, C1-C12 alkoxy, C1-C2 substituted alkoxy, C1-C3 substituted alkoxy, C1-C4 substituted alkoxy, C1-C5 substituted alkoxy, C1-C6 substituted alkoxy, C1-C7 substituted alkoxy, C1-C8 substituted alkoxy, C1-C9 substituted alkoxy, C1-C10 substituted alkoxy, C1-C11 substituted alkoxy or C1-C12 substituted alkoxy. In this case, for example, C1-C10 alkoxy refers to straight or branched chain alkoxy having 1-10 carbon atoms, including methoxy (CH₃O-), ethoxy (C₂H₅O-), n-propoxy (CH₃CH₂CH₂O-), etc.

The term "carbocyclic group" refers to a group having a cyclic structure containing only carbon, except for the aforementioned "cycloalkyl", "substituted cycloalkyl", "cycloalkenyl", and "substituted cycloalkenyl". A carbocyclic group may be an aromatic system or a non-aromatic system, and monocyclic or polycyclic. The term "substituted carbocyclic group" refers to a carbocyclic group in which one or more H are substituted with substituent(s) defined below. Specific examples may be C3-C4 carbocyclic group, C3-C5 carbocyclic group, C3-C6 carbocyclic group, C3-C7 carbocyclic group, C3-C8 carbocyclic group, C3-C9 carbocyclic group, C3-C10 carbocyclic group, C3-C11 carbocyclic group, C3-C12 carbocyclic group, C3-C4 substituted carbocyclic group, C3-C5 substituted carbocyclic group, C3-C6 substituted carbocyclic group, C3-C7 substituted carbocyclic group, C3-C8 substituted carbocyclic group, C3-C9 substituted carbocyclic group, C3-C10 substituted carbocyclic group, C3-C11 substituted carbocyclic group or C3-C12 substituted carbocyclic group. A carbocyclic group may be also a C4-C7 carbocyclic group or a C4-C7 substituted carbocyclic group. Examples of a carbocyclic group include a phenyl group, and the following compound from which one hydrogen atom is deleted: 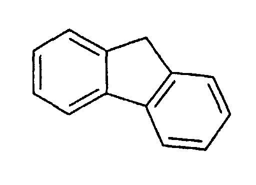 where the position of the deleted hydrogen atom may be any chemically possible position which may be present on an aromatic ring or a non-aromatic ring.

The term "heterocyclic group" refers to a group having a cyclic structure containing carbon and a hetero atom. In this case, the hetero atom is selected from the group consisting of O, S and N. The heterocyclic group may contain one or more hetero atoms which may be the same or different from one another. The heterocyclic group may be an aromatic system or a non-aromatic system, and may be monocyclic or polycyclic. The term "substituted heterocyclic group" refers to a heterocyclic group in which one or more H are substituted with substituent(s) defined below. Specific examples may be C3-C4 carbocyclic group, C3-C5 carbocyclic group, C3-C6 carbocyclic group, C3-C7 carbocyclic group, C3-C8 carbocyclic group, C3-C9 carbocyclic group, C3-C10 carbocyclic group, C3-C11 carbocyclic group, C3-C12 carbocyclic group, C3-C4 substituted carbocyclic group, C3-C5 substituted carbocyclic group, C3-C6 substituted carbocyclic group, C3-C7 substituted carbocyclic group, C3-C8 substituted carbocyclic group, C3-C9 substituted carbocyclic group, C3-C10 substituted carbocyclic group, C3-C11 substituted carbocyclic group or C3-C12 substituted carbocyclic group, in which one or more carbon atoms are replaced with hetero atom(s). The heterocyclic group may be a C4-C7 carbocyclic group or C4-C7 substituted carbocyclic group in which one or more carbon atoms are replaced with hetero atom(s). Examples of the heterocyclic group include thienyl, pyrrolyl, furyl, imidazolyl, pyridyl, etc. Preferable heterocyclic groups include the following compounds from which one hydrogen atom is deleted: 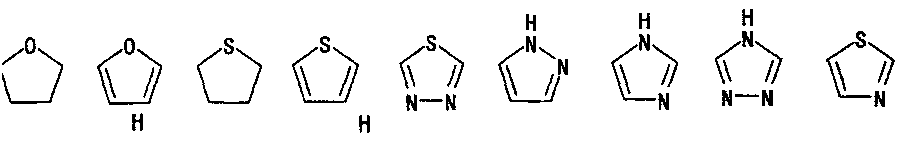 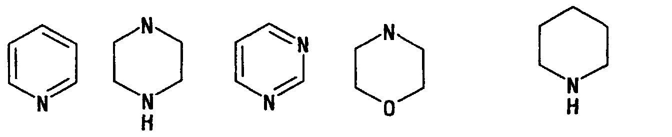 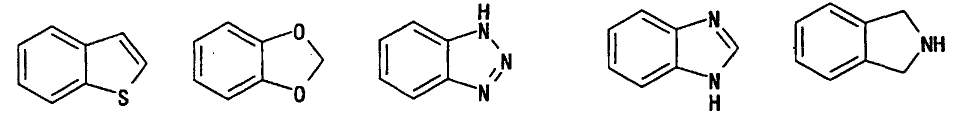 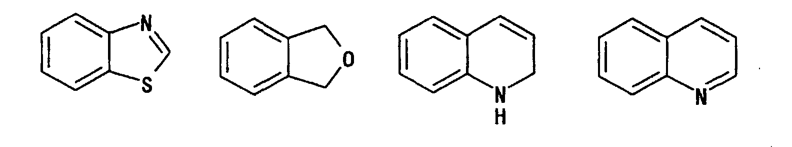 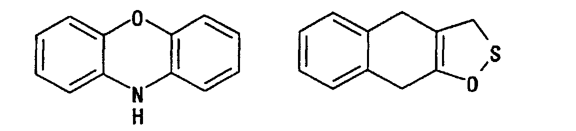 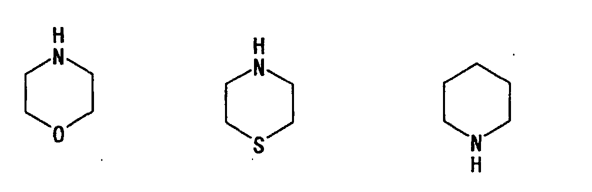 where the position of the deleted hydrogen atom may be any chemically possible position which may be present on an aromatic ring or a non-aromatic ring.

A carbocyclic group or heterocyclic group as used herein may be substituted with a monovalent substituent and, in addition, a divalent substituent defined below. Such a divalent substitution may be an oxo substitution (=0) or thioxo substitution (=S).

A substituted heterocyclic group may be the following, for example.

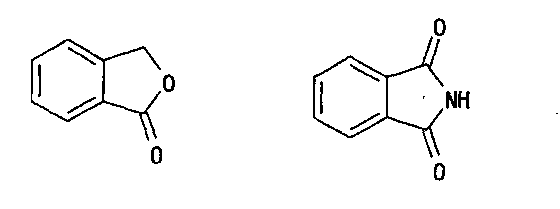

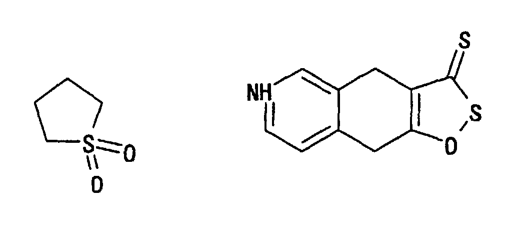

The term "halogen" refers to a monovalent group of an element belonging to the VII B group in the periodic table, such as, for example, fluorine (F), chlorine (Cl), bromine (Br), and iodine (I).

The term "hydroxy" refers to a group represented by -OH. The term "substituted hydroxy" refers to hydroxy in which one or more H are substituted with substituent(s).

The term "thiol" refers to a hydroxy group (mercapto group) in which the oxygen atom of the hydroxy group is replaced with a sulfur atom, and which is represented by -SH. The term "substituted thiol" refers to a mercapto group in which one or more H are substituted with substituent(s) defined below.

The term "cyano" refers to a group represented by-CN. The term "nitro" refers to a group represented by -NO₂. The term "amino" refers to a group represented by -NH₂. The term "substituted amino" refers to amino in which one or more H are substituted with substituent(s) defined below.

The term "carboxy" refers to a group represented by -COOH. The term "substituted carboxy" refers to carboxy in which one or more H are substituted with substituent(s) defined below.

The term "thiocarboxy" refers to a carboxy group in which one or both oxygen atoms of the carboxy group are substituted with a sulfur atom, and which is represented by -C(=S)OH, -C(=O)SH or -CSSH. The term "substituted thiocarboxy" refers to thiocarboxy in which one or more H are substituted with substituent(s) defined below.

The term "acyl" refers to a monovalent group which is obtained by removing OH from carboxylic acid. Representative examples of the acyl group include acetyl (CH₃CO-), benzoyl (C₆H₅CO-), etc. The term "substituted acyl" refers to acyl in which hydrogen is substituted with a substituent defined below.

The term "amide" refers to a group obtained by substituting hydrogen of ammonia with an acid radical (acyl group) , which is preferably represented by -CONH₂. The term "substituted amide" refers to amide which is substituted.

The term "carbonyl" refers to a generic term of what contains -(C=O)- which is the characteristic group of aldehydes and ketones. The term "substituted carbonyl" refers to a carbonyl group which is substituted with a substituent selected below.

The term "thiocarbonyl" refers to a carbonyl group in which the oxygen atom of the carbonyl group is substituted with a sulfur atom, and contains a characteristic group - (C=S)-. Thiocarbonyl includes thioketones and thioaldehydes. The term "substituted thiocarbonyl" refers to thiocarbonyl which is substituted with a substituent selected below.

The term "sulfonyl" refers to a generic term of what contains the characteristic group -SO₂-. The term "substituted sulfonyl" refers to sulfonyl which is substituted with a substituent selected below.

The term "sulfinyl" refers to a generic term of what contains the characteristic group -SO-. The term "substituted sulfinyl" refers to sulfinyl which is substituted with a substituent selected below.

The term "alkylthio" refers to an alkyl group coupled with a sulfur atom, which is generally represented by -S-R (where R is an alkyl group in which one hydrogen atom is deleted).

The term "arylthio" refers to an aryl group coupled with a sulfur atom, which is generally represented by -S-R (where R is an aryl group in which one hydrogen atom is deleted).

The term "aryl" refers to an aromatic hydrocarbon group in which one hydrogen atom bound to the ring is removed, and is herein included in the carbocyclic group.

Substitution refers to that one or more hydrogen atoms of a certain organic compound or substituent are substituted with other atom(s) or atomic group(s), unless otherwise mentioned. It is possible to remove one hydrogen atom to generate a monovalent substituent, and also to remove two hydrogen atoms to generate a divalent substituent. Substituents as used herein are preferably defined below.

When R¹ or R² is substituted,
R¹ is represented by R^{1A}-(R^{1B})ₙ and R² is represented by R^{2A}-(R^{2B})ₙ where R^{1A} and R^{2A} are separately (n+1)-valent groups in which n hydrogen atoms are removed from the respective R¹ and R²; and
R^{1B} or R^{2B} may be selected from the group consisting of alkyl, substituted alkyl, cycloalkyl, substituted cycloalkyl, alkenyl, substituted alkenyl, cycloalkenyl, substituted cycloalkenyl, alkynyl, substituted alkynyl, alkoxy, substituted alkoxy, carbocyclic group, substituted carbocyclic group, heterocyclic group, substituted heterocyclic group, halogen, hydroxy, substituted hydroxy, thiol, substituted thiol, cyano, nitro, amino, substituted amino, carboxy, substituted carboxy, acyl, substituted acyl, thiocarboxy, substituted thiocarboxy, amide, substituted amide, substituted carbonyl, substituted thiocarbonyl, substituted sulfonyl, and substituted sulfinyl.

When R^{1B} or R^{2B} is substituted,
R^{1B} is represented by R^{1C}-(R^{1D})ₙ and R^{2B} is represented by R^{2C}-(R^{2D})ₙ where R^{1C} and R^{2C} are separately (n+1)-valent groups in which n hydrogen atoms are removed from the respective R^{1B} and R^{2B}; and
R^{1D} or R^{2D} may be selected from the group consisting of alkyl, substituted alkyl, cycloalkyl, substituted cycloalkyl, alkenyl, substituted alkenyl, cycloalkenyl, substituted cycloalkenyl, alkynyl, substituted alkynyl, alkoxy, substituted alkoxy, carbocyclic group, substituted carbocyclic group, heterocyclic group, substituted heterocyclic group, halogen, hydroxy, substituted hydroxy, thiol, substituted thiol, cyano, nitro, amino, substituted amino, carboxy, substituted carboxy, acyl, substituted acyl, thiocarboxy, substituted thiocarboxy, amide, substituted amide, substituted carbonyl, substituted thiocarbonyl, substituted sulfonyl, and substituted sulfinyl.

When R^{1D} or R^{2D} is substituted,
R^{1D} is represented by R^{1E}-(R^{1F})ₙ and R^{2D} is represented by R^{2E}-(R^{2F})ₙ where R^{1E} and R^{2E} are separately (n+1)-valent groups in which n hydrogen atoms are removed from the respective R^{1D} and R^{2D}; and
R^{1F} or R^{2F} is selected from the group consisting of alkyl, substituted alkyl, cycloalkyl, substituted cycloalkyl, alkenyl, substituted alkenyl, cycloalkenyl, substituted cycloalkenyl, alkynyl, substituted alkynyl, alkoxy, substituted alkoxy, carbocyclic group, substituted carbocyclic group, heterocyclic group, substituted heterocyclic group, halogen, hydroxy, substituted hydroxy, thiol, substituted thiol, cyano,nitro, amino, substituted amino, carboxy, substituted carboxy, acyl, substituted acyl, thiocarboxy, substituted thiocarboxy, amide, substituted amide, substituted carbonyl, substituted thiocarbonyl, substituted sulfonyl, and substituted sulfinyl.

When R^{1F} or R^{2F} is substituted,
R^{1F} is represented by R^{1G}-(R^{1H})ₙ and R^{2F} is represented by R^{2G}-(R^{2H})ₙ where R^{1G} and R^{2G} are separately (n+1)-valent groups in which n hydrogen atoms are removed from the respective R^{1F} and R^{2F}; and
R^{1H} or R^{2H} may be selected from the group consisting of alkyl, substituted alkyl, cycloalkyl, substituted cycloalkyl, alkenyl, substituted alkenyl, cycloalkenyl, substituted cycloalkenyl, alkynyl, substituted alkynyl, alkoxy, substituted alkoxy, carbocyclic group, substituted carbocyclic group, heterocyclic group, substituted heterocyclic group, halogen, hydroxy, substituted hydroxy, thiol, substituted thiol, cyano, nitro, amino, substituted amino, carboxy, substituted carboxy, acyl, substituted acyl, thiocarboxy, substituted thiocarboxy, amide, substituted amide, substituted carbonyl, substituted thiocarbonyl, substituted sulfonyl, and substituted sulfinyl.

When R^{1H} or R^{2H} is substituted, this substitution may be conducted in a manner similar to that for R^{1F} or R^{2F}. The subsequent substituents may be substituted in a similar manner.

Needless to say, the above-described number n is not always the same among the substitutents and may be separately selected for each substituent. When n is greater than or equal to 2, each substituent represented by () n may be the same or different from one another.

In one embodiment, examples of preferable substituents for R¹ and R² include methyl, ethyl, n-butyl, benzyl, -OCOCH₃, or the following groups.

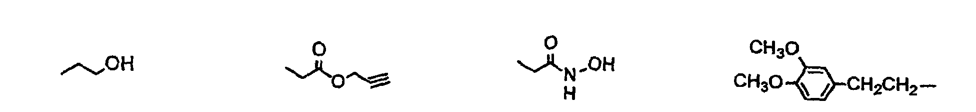

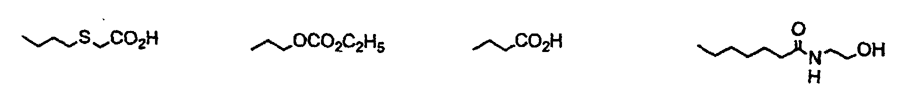

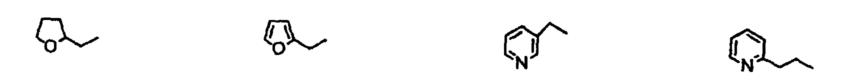

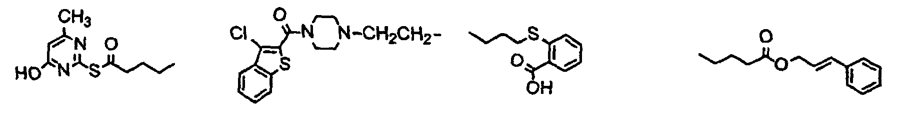

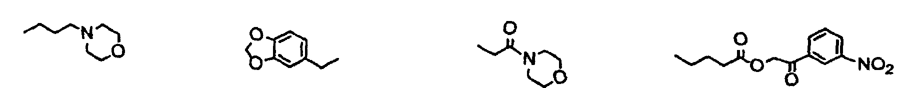

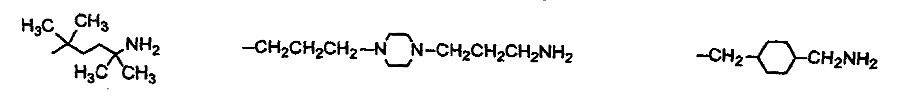

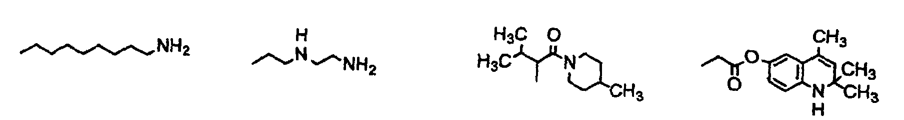

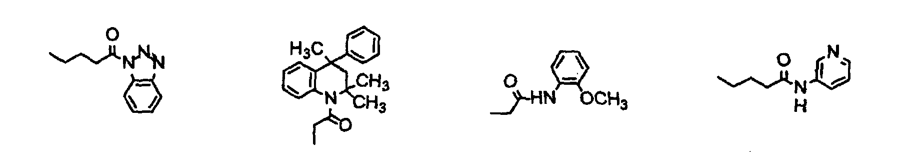

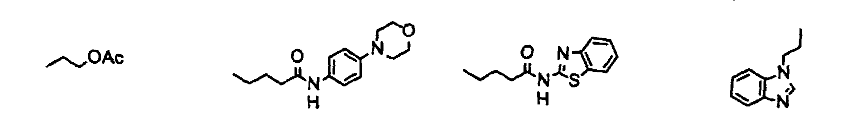

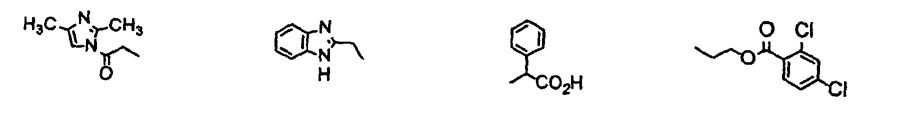

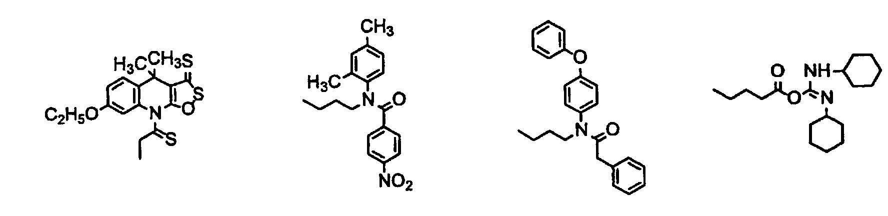

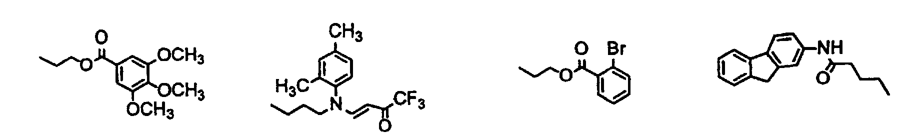

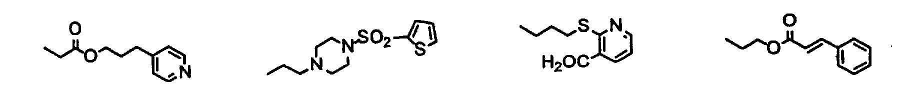

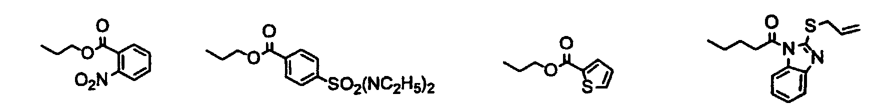

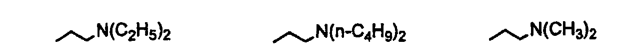

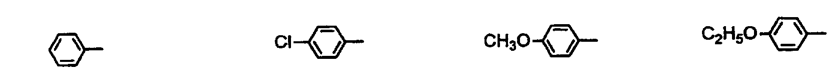

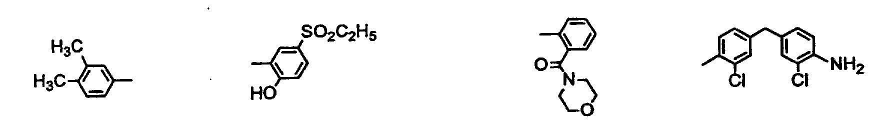

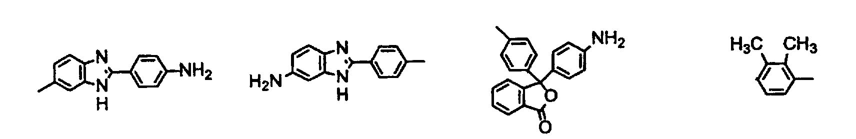

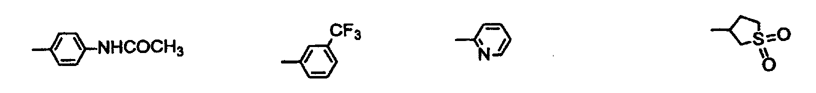

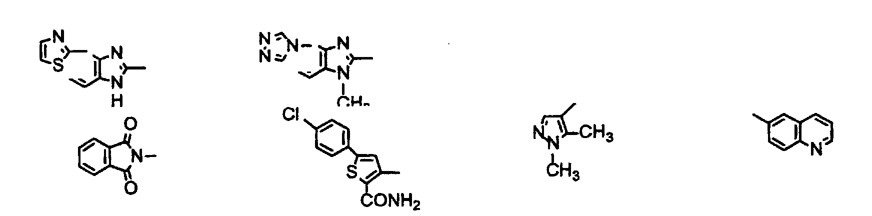

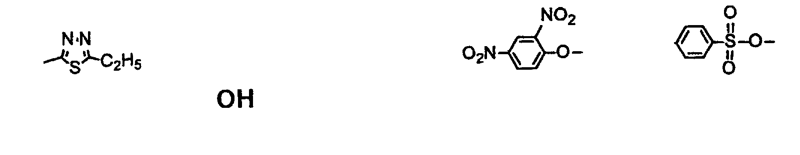

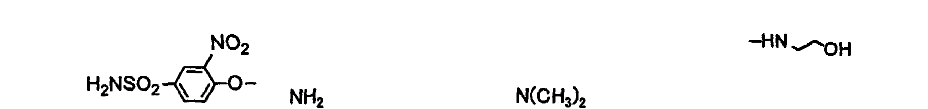

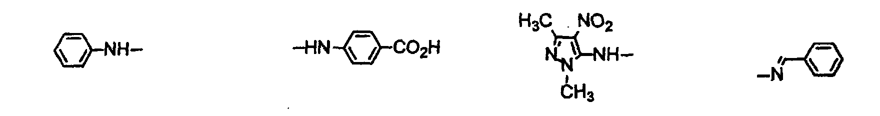

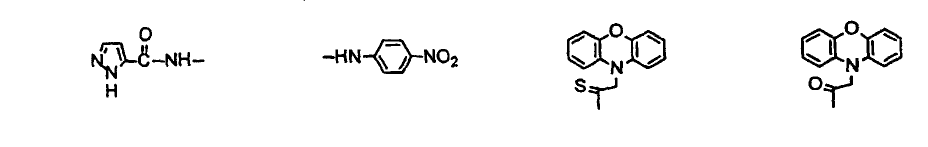

X¹, X², Y¹ and Y² may be each separately any of the above-described substitutents. Preferably, X¹, X², Y¹ and Y² may be separately selected from the group consisting of hydrogen, halogen, alkoxy, substituted alkoxy, amino, substituted amino, alkylthio, substituted alkylthio, arylthio, substituted arylthio, nitro, carboxy, substituted carboxy, acyl, substituted acyl, and substituted sulfonyl. When X¹, X², Y¹ or Y² is substituted, examples of the substituent include the above-described R^{1B} or R^{2B}, etc. More preferably, X¹, X², Y¹ and Y² each separately have a group selected from the group consisting of hydrogen, -Cl, -Br, -OCH₃, -OCF₃, -N(CH₃)₂, -NH₂, -SC₂H₅, -S(n-propyl), -NO₂, -CO₂H, -SCH₂CH₂OH, -SPh, SO₂N(CH₂CH₂OH)₂, COCH₃, COPh (wherein Ph is a phenyl group),

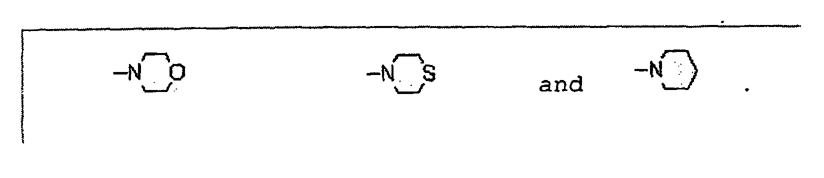

X¹ and X², or Y¹ and Y² can also be coupled together to form a ring to generate carbocyclic group, substituted carbocyclic group, heterocyclic group, or substituted heterocyclic group.

In a more preferable embodiment of the compound of the present invention, R¹ and R² may be separately any of the following substituents. Preferably, R¹ and R² are simultaneously any of the following substituents.

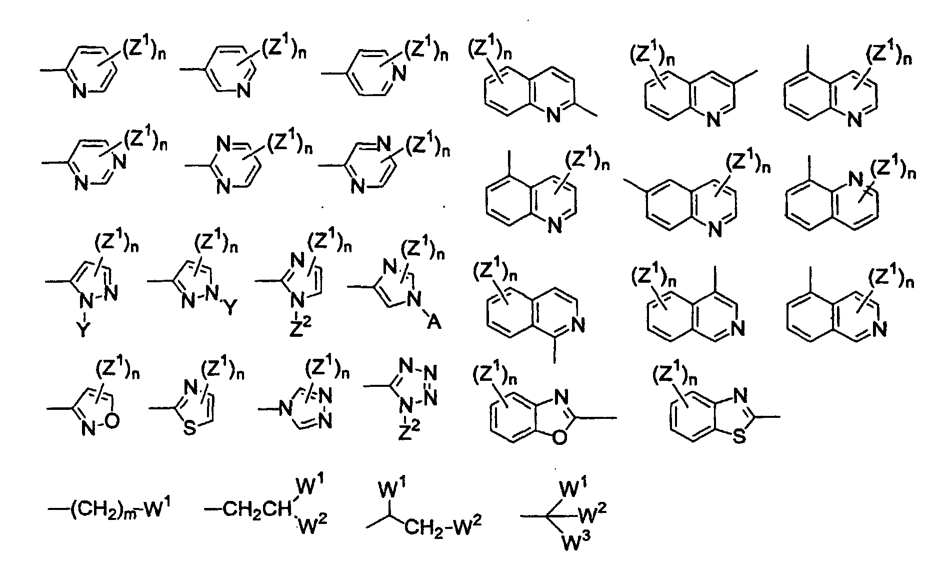

In the above-described substituents, Z¹ is selected from the group consisting of hydrogen, alkyl, substituted alkyl, halogen, hydroxy, substituted hydroxy, alkoxy, substituted alkoxy, acyl, substituted acyl, carbocyclic group, substituted carbocyclic group, heterocyclic group, substituted heterocyclic group, amino, substituted amino, nitro, cyano, carboxy, substituted carboxy, amide, and substituted amide.
Z² is selected from the group consisting of hydrogen, alkyl, carbocyclic group, substituted carbocyclic group, heterocyclic group, and substituted heterocyclic group.
n is an integer from 1 to 4.
W¹, W² and W³ are separately selected from the group consisting of hydrogen, alkyl, substituted alkyl, halogen, hydroxy, substituted hydroxy, alkoxy, substituted alkoxy, acyl, substituted acyl, carbocyclic group, substituted carbocyclic group, heterocyclic group, substituted heterocyclic group, amino, substituted amino, nitro, cyano, carboxy, substituted carboxy, amide, and substituted amide. Further, two substituents may be coupled together to generate imino, substituted imino, hydroxyimino, and alkylimino.
m is an integer greater than or equal to 1.

In an even more preferable embodiment of the compound of the present invention, R¹ and R² may be separately any of the following substituents.

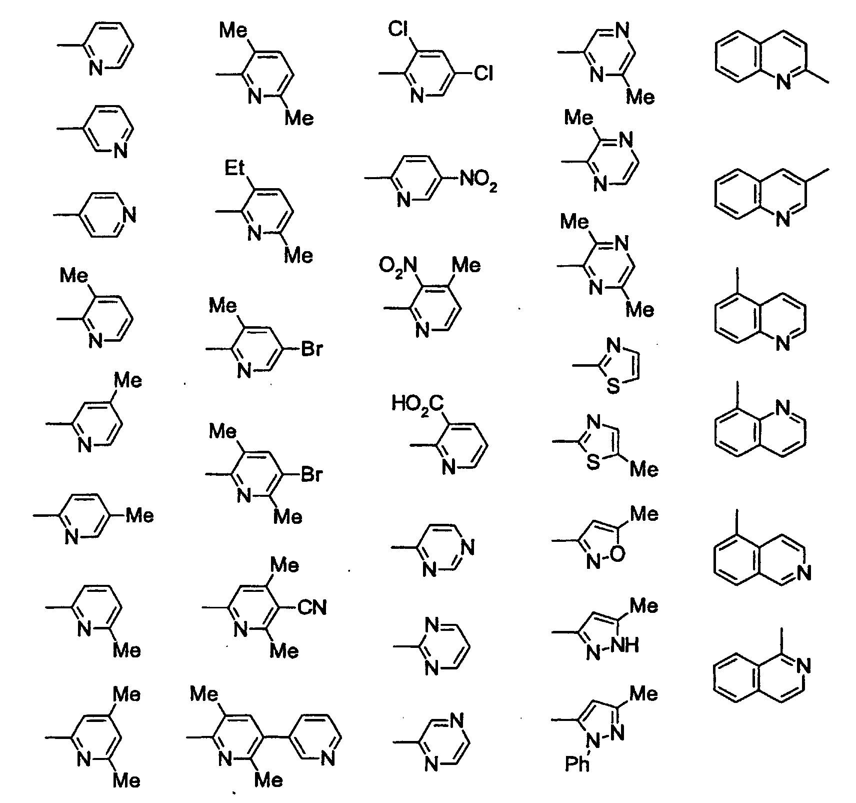

In another preferable embodiment of the compound of the present invention, R¹ and R² may be separately any of the following substituents.

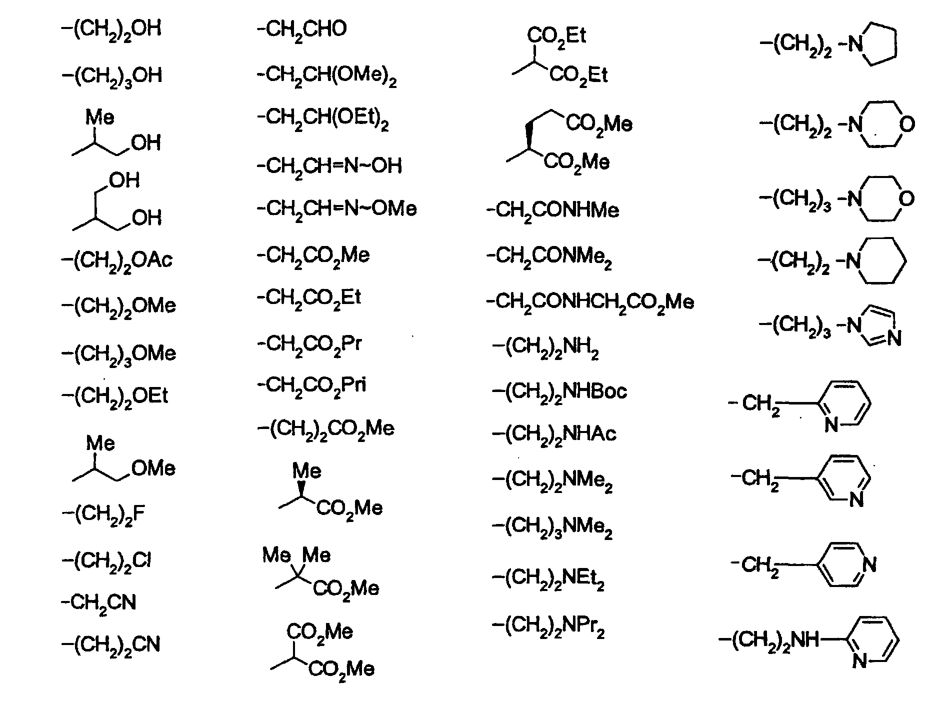

In a more preferable embodiment of the compound of the present invention, R¹ and R² may be separately any of the following substituents.

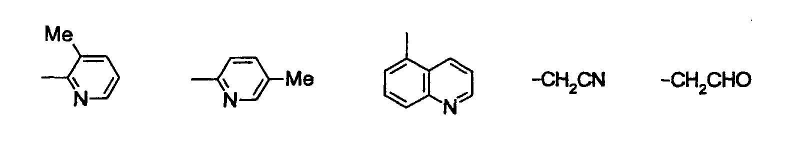

In another embodiment, R¹ and R² may be separately any of the following substituents.

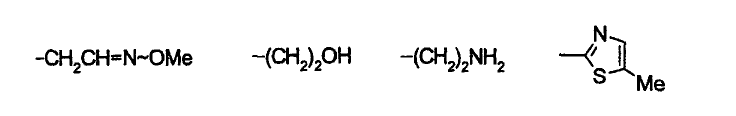

In another embodiment, R¹ and R² may be separately any of the following substituents.

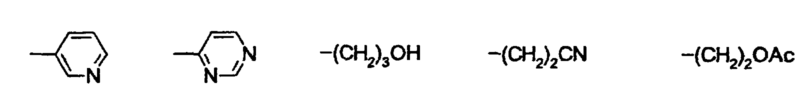

In another embodiment, R¹ and R² may be separately any of the following substituents.

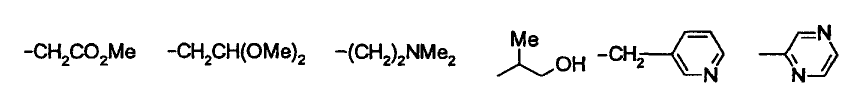

In another embodiment, R¹ and R² may be separately any of the following substituents.

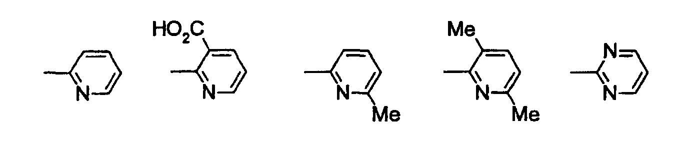

In another embodiment, R¹ and R² may be separately any of the following substituents.

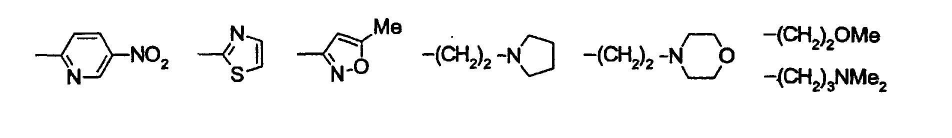

The compound of the present invention may be synthesized with a combination of known methods in the art. Examples of such methods include, but are not limited to, the following reaction routes.

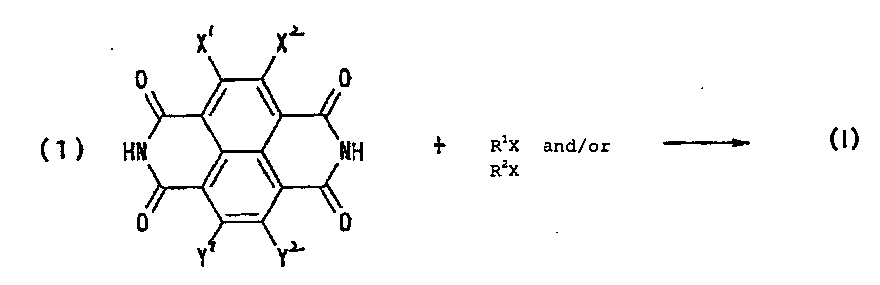

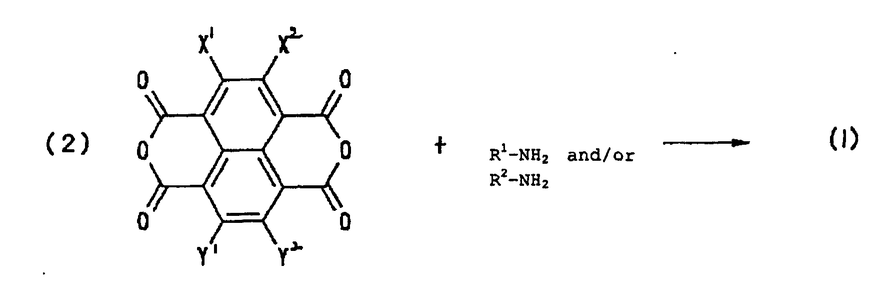

Note that X¹, X², Y¹ and Y² may be appropriately introduced by a method well known to those skilled in the art after the basic structure of compound (I) is constructed in accordance with the above-described reaction. In this case, R¹-NH₂ and/or R²-NH₂ can be subjected to the reaction, in the presence or absence of AcOH, in dimethylsulfoxide (DMSO), N,N-dimethylformamide (DMF), N,N-dimethylacetoamide (DMA),1-methyl-2-pyrrolidone (NMP), 1,3-dimethyl-2-imidazolidinone (DMI). When the salts of R¹-NH₂ and/or R³-NH₂ are used as a starting material, the reaction may be conducted in the presence of a base, such as, for example, sodium acetate and potassium acetate.

The term "bacterial eradication" as used herein generally refers to that a certain bacterium is reduced, or substantially or perfectly eradicated from a certain tissue or organ, or a partial region thereof. This activity is called "bacterial eradication activity". In one embodiment herein, bacterial eradication or bacterial eradication activity is eradication of a bacterium of the genus Helicobacter. Preferably, bacterial eradication or bacterial eradication activity is H, pylori eradication. Bacterial eradication activity can be measured with an in vitro or in vivo assay. In one embodiment, bacterial eradication activity can be evaluated by the following method: a subject sample, such as a pharmaceutical agent or a candidate compound, is administered to a H. pylori infection mouse model and, thereafter, the number of bacteria in the digestive organs, such as the stomach or the duodenum, is counted. Assays for bacterial eradication activity are illustrated and exemplified in the Examples section (below) of the present specification.

The compound of the present invention may be used alone. The compound of the present invention may be used in conjunction with another medicament or excipient. Any medicament that is used in conjunction with a conventional anti-Helicobacter agent can be employed in conjunction with the compound of the present invention, for example. Specifically, examples of such a medicament include, but are not limited to, antibacterial agents, mucosal protection agent promoters, anti-gastrin agents, H₂-receptor antagonists, proton pump inhibitors, bismuth preparations, and gastrointestinal drugs. Any antibacterial agent that is used in conjunction with a conventional anti-Helicobacter agent can be employed in conjunction with the compound of the present invention. Specifically, examples of such an antibacterial agent include, but are not limited to, conventional native or synthetic antibiotics (e.g., penicillin antibiotics, cephem antibiotics, tetracycline antibiotics, aminoglycoside antibiotics, chloramphenicol, polypeptide antibiotics, macrolide antibiotics, and polyene antibiotics, etc.), antifungal agents, antiviral agents, sulfa agents or quinolone antibacterial agents. Any mucosal protection agent promoter that is used in conjunction with a conventional anti-Helicobacter agent can be employed. Specifically, examples of the mucosal protection agent promoter include, but are not limited to, benexate hydrochloride, plaunotol, and sofalcone. Any anti-gastrin agent that is used in conjunction with a conventional anti-Helicobacter agent can be employed. Specifically, examples of the anti-gastrin agent include, but are not limited to, proglumide and oxethazaine. Any anti-H₂ receptor antagonist that is used in conjunction with a conventional anti-Helicobacter agent can be employed. Specifically, examples of the anti-H₂ receptor antagonist include, but are not limited to, cimetidine, famotidine, and ranitidine. Any proton pump inhibitor that is used in conjunction with a conventional anti-Helicobacter agent can be employed. Specifically, examples of the proton pump inhibitor include, but are not limited to, omeprazole, lansoprazole, pantoprazole, pariprazole, and leminoprazole. Any bismuth preparation that is used in conjunction with a conventional anti-Helicobacter agent can be employed. Specifically, examples of the bisumth preparation include, but are not limited to, colloidal bismuth, bismuth subsalicylate, and bismuth subnitrate. Any gastrointestinal drug that is used in conjunction with a conventional anti-Helicobacter agent can be employed. Specifically, examples of the gastrointestinal drug include, but are not limited to, various pharmaceutical agents which exhibit an antacid, analgesic, or stomachic effect.

The compound of the present invention specifically acts on a bacterium of the genus Helicobacter. A problem with administration of a conventional antibiotic is that benign bacteria in the digestive tract are killed. The compound of the present invention can reduce or avoid such a problem, whereby side effects due to the destruction of benign bacteria can be reduced or avoided.

The compound or composition of the present invention (even when used alone) may be sufficiently effective as an anti-Helicobacter agent as specifically acting on a bacterium of the genus Helicobacter. If the compound or composition of the present invention is used alone, there is no need to worry about the side effects caused by other medicaments which are conventionally used in conjunction with an anti-Helicobacter agent. Specifically, when an antibacterial agent, such as, for example, penicillin antibiotics, macrolide antibiotics, tetracycline antibiotics, and nitroimidazole and quinolone antibacterial agents, is used in conjunction, side effects (e.g, diarrhea, etc.) which kill benign bacteria within the digestive organs , have been reported. Concerns about such side effects no longer exist. This effect is not achieved by conventional antibiotics, and is one of the advantageous effects of the present invention.

The composition of the present invention can treat various diseases associated with bacteria of the genus Helicobacter. The term "diseases caused by Helicobacter" as used herein refers to diseases or disorders, or medical conditions in which a bacterium of the genus Helicobacter is directly or indirectly involved. Examples of the diseases caused by Helicobacter include digestive organs diseases. Preferably, the composition of the present invention is useful for treatment of gastric ulcers, duodenal ulcers, or gastritis and further other diseases caused by Helicobacter (e.g., other digestive organs diseases, such as, for example, gastric cancer and dyspepsia).

Examples of the target pathogens of the composition of the present invention include bacteria of the genus Helicobacter. A particular target Helicobacter of the composition of the present invention may be Helicobacter pylori or Helicobacter felis.

The term "formulation" as used herein refers to a preparation which contains the compound of the present invention for treatment with a drug. A formulation can be produced in various amounts or forms by people involved in medication or pharmaceutical production (e.g., medical practitioners, etc.) under various circumstances of treatment, prevention, or avoidance of recurrence.

The present invention provides a method for treating, curing, and/or preventing diseases caused by Helicobacter and/or avoiding the recurrence of such diseases by administering to a subject the compound of the present invention or a pharmaceutically acceptable salt or hydrate thereof, or a pharmaceutical composition containing the same. In this case, the subject may be a patient with a disease caused by Helicobacter or a subject who is suspected of suffering from the disease or is likely to suffer from the disease or its recurrence in the future.

The dosage can be generally determined with reference to the up-to-date Japanese Pharmacopoeia, American Pharmacopoeia, or equivalents thereof in other countries. The dosage includes, but is not limited to, intracutaneous, intramuscular, intraperitoneal, intravenous, subcutaneous, intranasal, extradural, and oral administration. The compound, composition, composition or formulation of the present invention can be administered via any convenient route (e.g., infusion or bolus injection, absorption via epithelium or the inner membrane of mucosa skin (e.g., oral mucosa, rectal mucosa, and intestinal mucosa, etc.), and may be administered in conj unction with another biologically active pharmaceutical agent.

Oral preparations are in various dosage forms, such as, for example, solid preparations (e.g., tablet, capsule, granule, powder, etc.) and liquid preparations (e.g., syrup, solution, suspension, etc.). Parenteral preparations may be used as solutions for injection (e.g., intravenous, intramuscular, subcutaneous injection, etc.) or suspension, or alternatively, percutaneous preparations (e.g., ointment, etc.) and parenteral preparations (e.g., suppository, etc.) .

The dose of the compound of the present invention depends on the age, weight and conditions of a subject or a method of administer the compound. Particularly, the dose is typically, but not limited to, 0.01 mg to 10 g for an adult in a day, and preferably 0.1 mg to 1 g, 1 mg to 100 mg, 0.1 mg to 10 mg, etc. in the case of oral administration; or 0.01 mg to 1 g, and preferably 0.01 mg to 100 mg, 0.1 mg to 100 mg, 1 mg to 100 mg, 0.1 mg to 10 mg, etc. in the case of parenteral administration.

As described above, the details of the present invention are provided in various forms. However, particular embodiments and examples as described herein are only for illustration purposes. It should be understood that the scope of the present invention is not limited at all to the particular illustrative embodiments and examples.

### Examples

### (Example 1: production of compound 2)

### (Synthesis of N,N'-bis-(2-pyridyl)naphthalene-1,4,5,8-tetracarboxylat e diimide(2))

11.29 g (120.0 mmol) of 2-aminopyridine and 25.4 ml (200.0 mmol) of chlorotrimethylsilane were added to 40 ml of DMF solution containing 10.73 g (40.0 mmol) of naphthalene-1,4,5,8-tetracarboxylic dianhydride (**1**), followed by heating at 160°C for 6 hours. The mixture was allowed cool to room temperature. Solid substance was isolated by filtering and washed by ethyl acetate, followed bydrying. The resultant solid substance (6.36 g) was washed by addition of 20 ml of 1N aqueous sodium hydroxide solution. This washing procedure was repeated four times, followed by recrystallization with DMSO:water. As a result, compound **2** (0.78 g) was obtained.

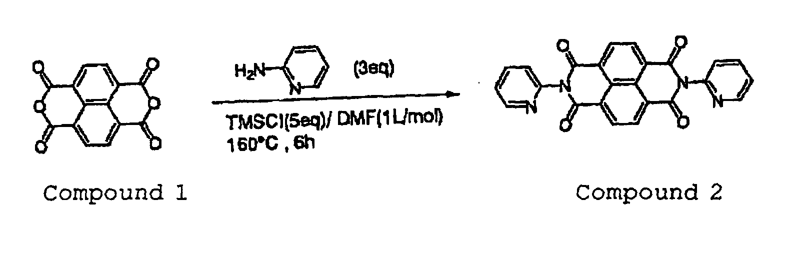

The physical data are the following.

Melting point (mp) >300°C. Note: 2000-5041-024-01. ¹H-NMR(DMSO-d6) δ ppm: 7.60 (ddd, J=7.8, 4.9, 1.0, 2H), 7.67 (dd, J=7.8, 1.0, 2H), 8.10 (ddd, J=7.8, 7.8, 2.0, 2H), 8.68-8.70 (m, 2H), 8.76 (s, 4H).

### (Example 2: in vitro test)

Firstly, the thus-synthesized compound of the present invention (2) was studied for its activity in vitro. For antibacterial activity, an agar plate dilution method was employed. The agar plate dilution method is well known in the art.

Specifically, a test compound containing the compound of the present invention was dissolved into dimethylsulfoxide, and serially diluted by two fold each time with sterile distilled water to prepare an antibacterial activity sample for the test compound containing the compound of the present invention. A BHI (Brain Heart Infusion) agar with 7% horse blood was used as medium for investigation of the antibacterial activity against H. pylori and H. felis. 1 ml of the sample of the compound of the present invention was added to 9 ml of the medium, followed by mixing to prepare an agar plate for measuring the antibacterial activity. MH (Mueller Hinton) agar medium was used to investigate the antibacterial activities against Pseudomonas aeruginosa, Escherichia coli, Klebsiella pneumoniae, and Staphylococcus aureus; and 10% horse serum-added MH agar medium was used to investigate the antibacterial activities against Streptococcus pneumoniae and Enterococcus faecalis. In both cases, agar plates for measuring the antibacterial activities are prepared in a manner similar to that for H. pylori and H. felis. For H. pylori and H. felis out of the bacteria to be subjected to the tests , 1 ml of each frozen bacterium stock solution was inoculated to 9 ml of 7% bovine fetal serum-added BHI liquid medium, and cultured at 37°C for 48 hours in a 10% CO₂ incubator. Each culture bacterium solution was prepared with the same medium to 10⁶ CFU/ml (colony formation unit/milliliter). 5 µl of the prepared bacterium solution was inoculated to the antibacterial activity measuring agar medium, and cultured at 37°C for 4 days in a 10% CO₂ incubator. MH liquid medium was used for Pseudomonas aeruginosa, Escherichia coli, Klebsiella pneumoniae, and Staphylococcus aureus; and 10% horse serum-added MH liquid medium was used for Streptococcus pneumoniae and Enterococcus faecalis. In both cases, 1 ml of each frozen bacterium stock solution was inoculated to 9 ml of the medium, and cultured at 37°C overnight. Each culture bacterium solution was prepared with the same medium as that had been used in the incubation to 10⁶ CFU/ml. 5 µl of the prepared bacterium solution was inoculated to the antibacterial activity measuring agar medium, and cultured at 37°C overnight.

The antibacterial activity of the compound against a subject bacterium (MIC: Minimum Inhibitory Concentration) was a maximum dilution ratio at which the growth of inoculated bacteria was not observed by the unaided eye.

The results are represented by MIC (Minimum Concentration).

**Table 1**

| Compound \ Bacterium | MIC (µg/ml) | | | | |
|---|---|---|---|---|---|
| | Compound 2 | Amoxicillin (AMPC) | Metronidazole (MNI) | Clarithromycin (CAM) | Tetracycline (TC) |
| Helicobacter pylori ATCC 43054 | 0.2 | 0.025 | >25.0 | 0.025 | 0.39 |
| Helicobacter pylori ATCC 43629 | 0.2 | 0.1 | 3.13 | 0.1 | 0.39 |
| Helicobacter pylori SR-9043 | 0.39 | 0.39 | >25.0 | >25.0 | 0.39 |
| Helicobacter pylori SS1 | 0.1 | 0.39 | 0.78 | 0.025 | No test |
| Helicobacter felis ATCC 49179 | 0.1 | 0.1 | 0.78 | <0.025 | 0.025 |
| Pseudomonas aeruginosa ATCC 25619 | >25.0 | >25.0 | >25.0 | >25.0 | >25.0 |
| Escherichia coli NIHJ JC-2 | >25.0 | 6.25 | >25.0 | 12.5 | 1.56 |
| Klebsiella pneumoniae ATCC 27736 | >25.0 | 0.2 | >25.0 | >25.0 | 3.13 |
| Staphylococcus aureus FDA 209P | >25.0 | 0.05 | >25.0 | 0.05 | 0.39 |
| Strepcococcus pneumoniae SR-1003 | >25.0 | 0.05 | >25.0 | 0.0125 | 0.39 |
| Enterococcus faecalis ATCC 19433 | >25.0 | 0.39 | >25.0 | >25.0 | >25.0 |

According to the results of this example, the antibacterial spectrum of one compound of the present invention is selective to bacteria of the genus Helicobacter. Specifically, the MIC against H. pylori was 0.1 µg/ml to 0.39 µg/ml. Further, this compound did not show cross-resistance against the pharmaceutical agents, such as, for example, amoxicillin, clarithromycin, and metronidazole.

Thereafter, other compounds of the present invention were similarly studied on MIC. The results are summarized in Table 2 below. Note that the structures of the compounds are the following.

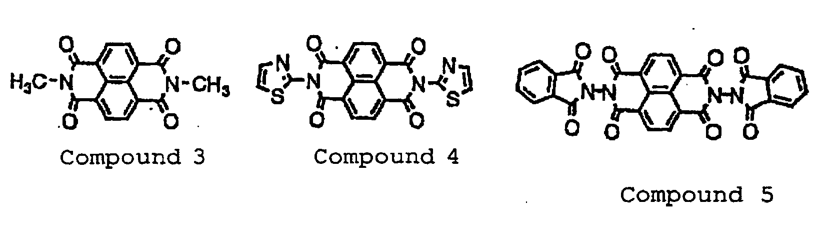

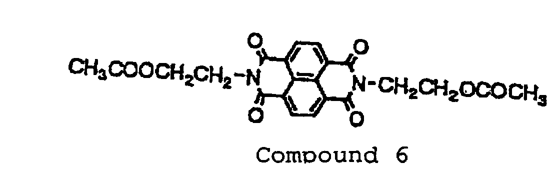

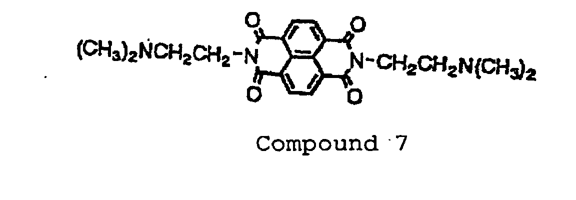

**Table 2**

| Compound \ Bacterium | MIC (µg/ml) | | | | |
|---|---|---|---|---|---|
| | Compound 3 | Compound 4 | Compound 5 | Compound 6 | Compound 7 |
| Helicobacter pylori ATCC 43054 | 6.25 | 2.5 | 12.5 | 0.31 | 0.39 |
| Pseudomonas aeruginosa ATCC 25619 | >25.0 | >25.0 | >25.0 | >25.0 | >25.0 |
| Escherichia coli NIHJ JC-2 | >25.0 | >25.0 | >25.0 | >25.0 | >25.0 |
| Staphylococcus aureus FDA 209P | >25.0 | >25.0 | >25.0 | >25.0 | >25.0 |

As can be seen from Table 2 above, all of the compounds of the present invention are effective for H. pylori, but none of them have any effect on the other bacteria at the tested concentrations. Therefore, the compound of the present invention is considered to be a H. pylori selective antibacterial agent.

### (Example 3: in vivo test for the compound of the present invention)

Next, the compound of the present invention was studied as to whether or not it had bacterial eradication activity in vivo. 5-week old mice of the ICR strain from CLEA Japan, Inc. were used. These mice were infected with H. pylori ATCC 43504 (day 0). After the mice had been confirmed to be infected with H. pylori, the dose indicated in Table 3 was administered into the mice twice a day (AM 9 and PM 5) from day 20 to day 22. At day 23 which was the day after the last day of the administration, the mice were dissected and the number of bacteria within their stomachs were counted. The bacteria count measurement was conducted in the following manner. Phosphate buffer was added to the collected stomachs to prepare homogenates. These homogenates were inoculated to 7% horse blood-added agar medium to which various drugs were added in order to suppress the growth of bacteria other than the genus Helicobacter, and cultured at 37°C for 7 days in a 10% CO₂ incubator. The bacterial eradication activity was judged by detecting the presence or absence of the growth of the bacteria. Table 3 shows the test conditions and results.

**Table 3**

| Compound | Dose (mg/kg) | Number of samples | Bacterial eradication number (bacterial eradication %) | Number of each bacterium in stomach (log CFU/stomach) |
|---|---|---|---|---|
| Compound **2** | 20 | 3 | 3(100) | <1.3,<1.3, <1.3 |
| Amoxicilli n (AMPC) | 20 | 3 | 1(33) | 1.60,<1.30, <1.3 |
| | 40 | 3 | 2(67) | <1.3,<1.3, 1.78 |
| Clarithrom ycin (CAM) | 20 | 3 | 0(0) | 2.79,2.89, 3.37 |
| | 40 | 3 | 0(0) | 1.6,1.6, 2.38 |
| Control | Only vehicle | 2 | 0(0) | 3.64,4.28 |
| Note: dose: twice a day with the indicated value (9 o'clock and 17 o'clock), oral administration for consecutive three days Limit of detection: 20(log.1.3)CFU/stomach | | | | |

According to the results of the in vivo tests, when a dose of 20 mg/kg of the compound of the present invention (compound 2) was administered, the bacterial eradication effect was recognized in all samples (3/3). When amoxicillin and clarithromycin were used as subject pharmaceutical agents , the bacterial eradication effect was not observed even in the case of 40 mg/kg administration.

### (Example 4: various synthetic examples)

Various compounds of the present invention were further synthesized in accordance with the method described in Example 1. The melting points and ¹H-NMR of some of the synthesized compounds were measured. NMR was conducted under the following conditions:

¹H-NMR values were measured in a solvent, i.e., deuterodimethylsulfoxide (DMSO-d₆), deuterochloroform (CDCl₃), deuteropyridine (pyridine-d₅), or deuterotrifluoroacetic acid (CF₃COOD) where tetramethylsilane was used as an internal standard. δ value is denoted by ppm. Coupling constant (J) is denoted by Hz. In the data, s means singlet, d means doublet, t means triplet, q means quartet, quint means quintet, sext means sextet, m means multiplet, and br means broad peak.

The compounds here synthesized have the following general formula.

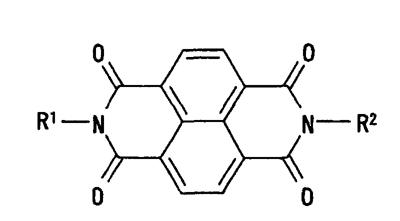

R¹ and R² are shown in Table 4 below. The measured melting point and NMR results of each compound are shown.

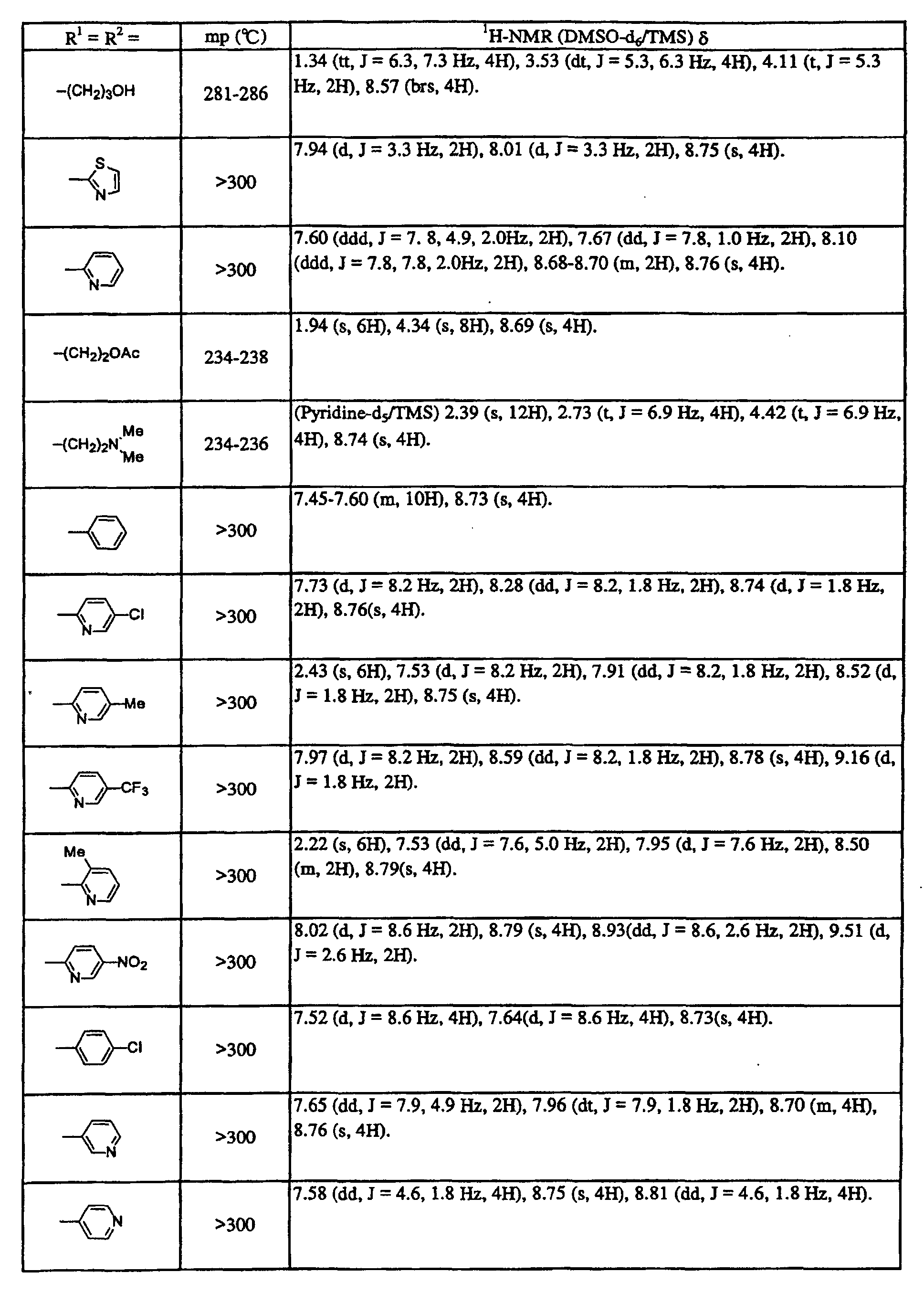

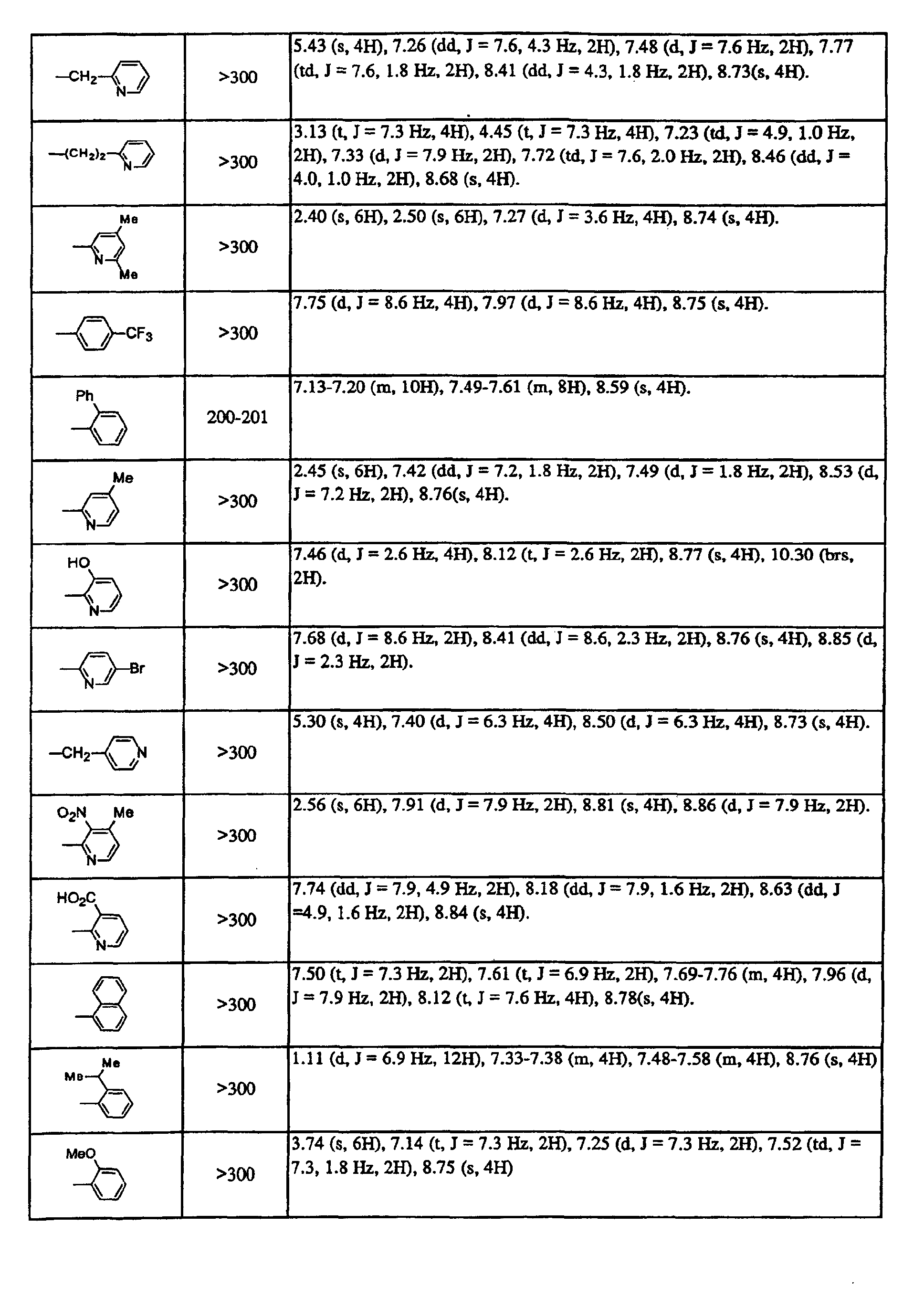

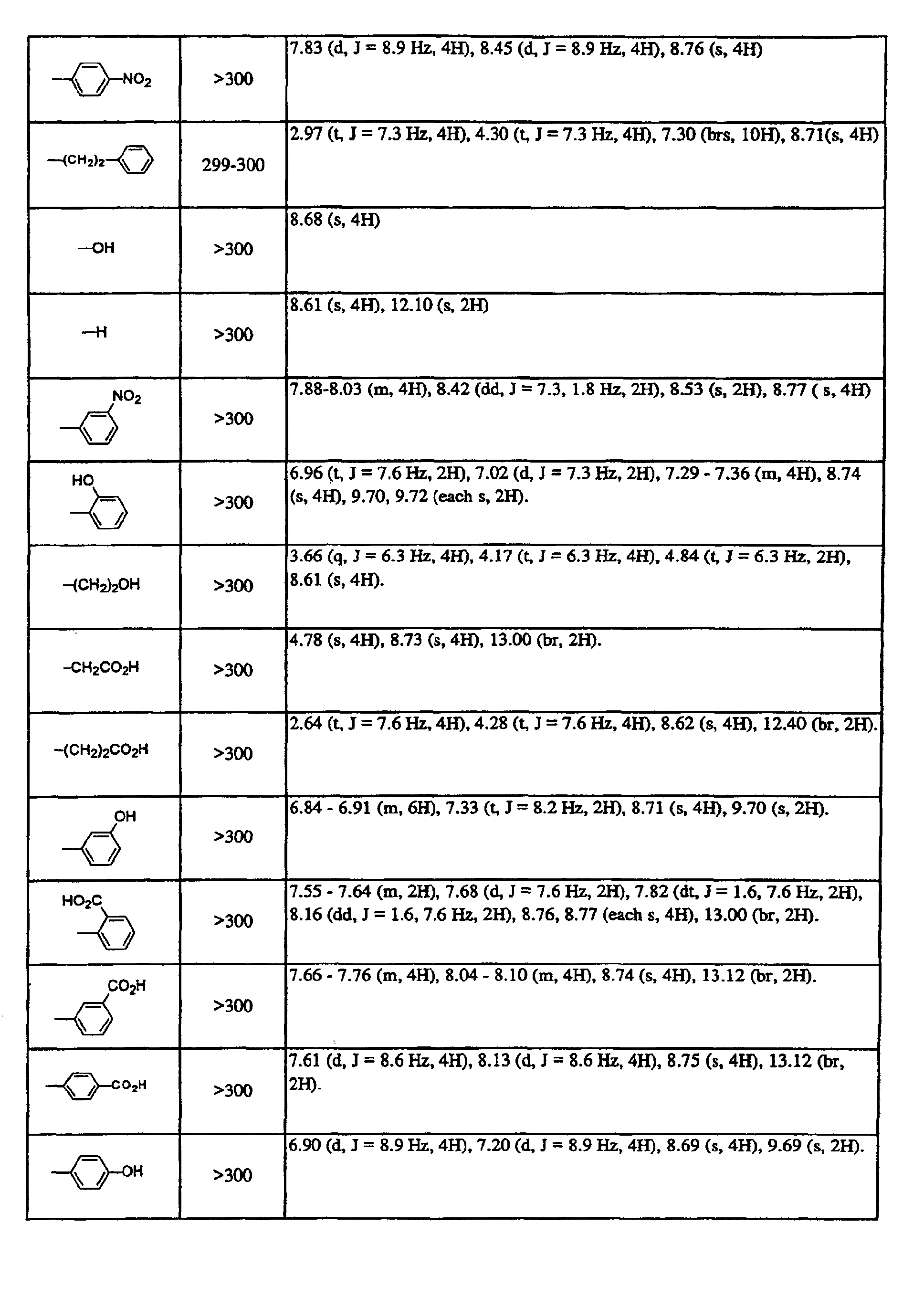

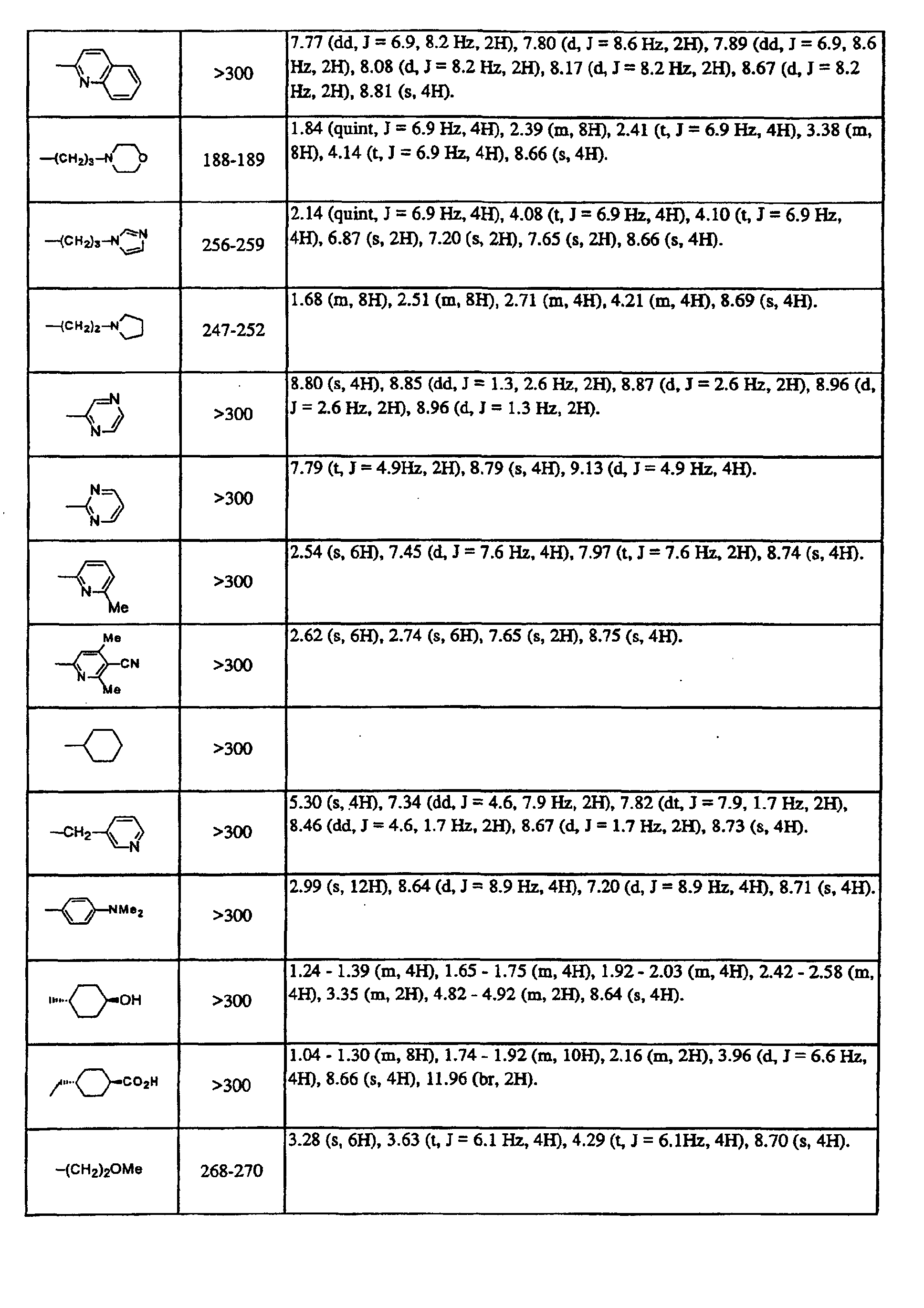

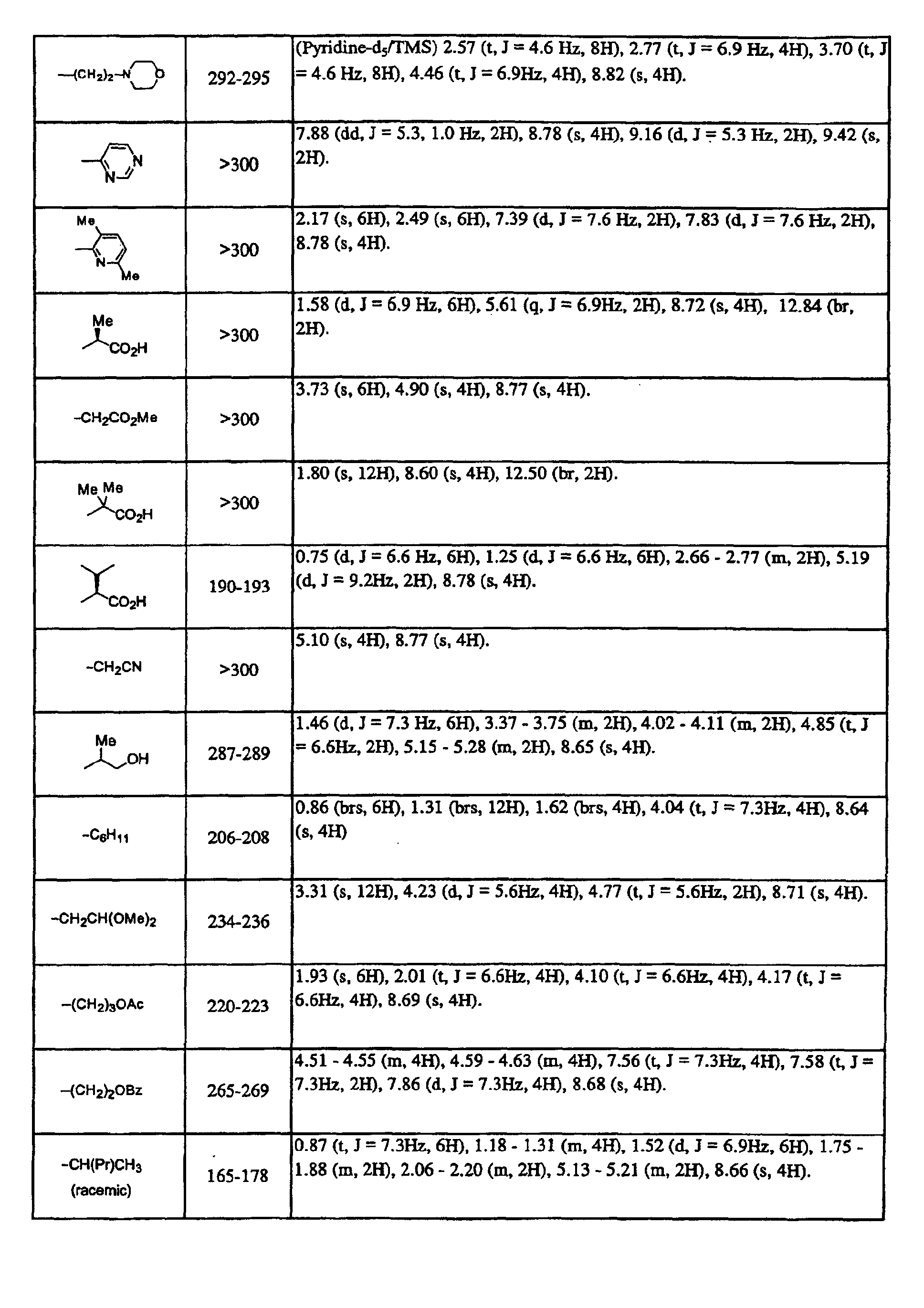

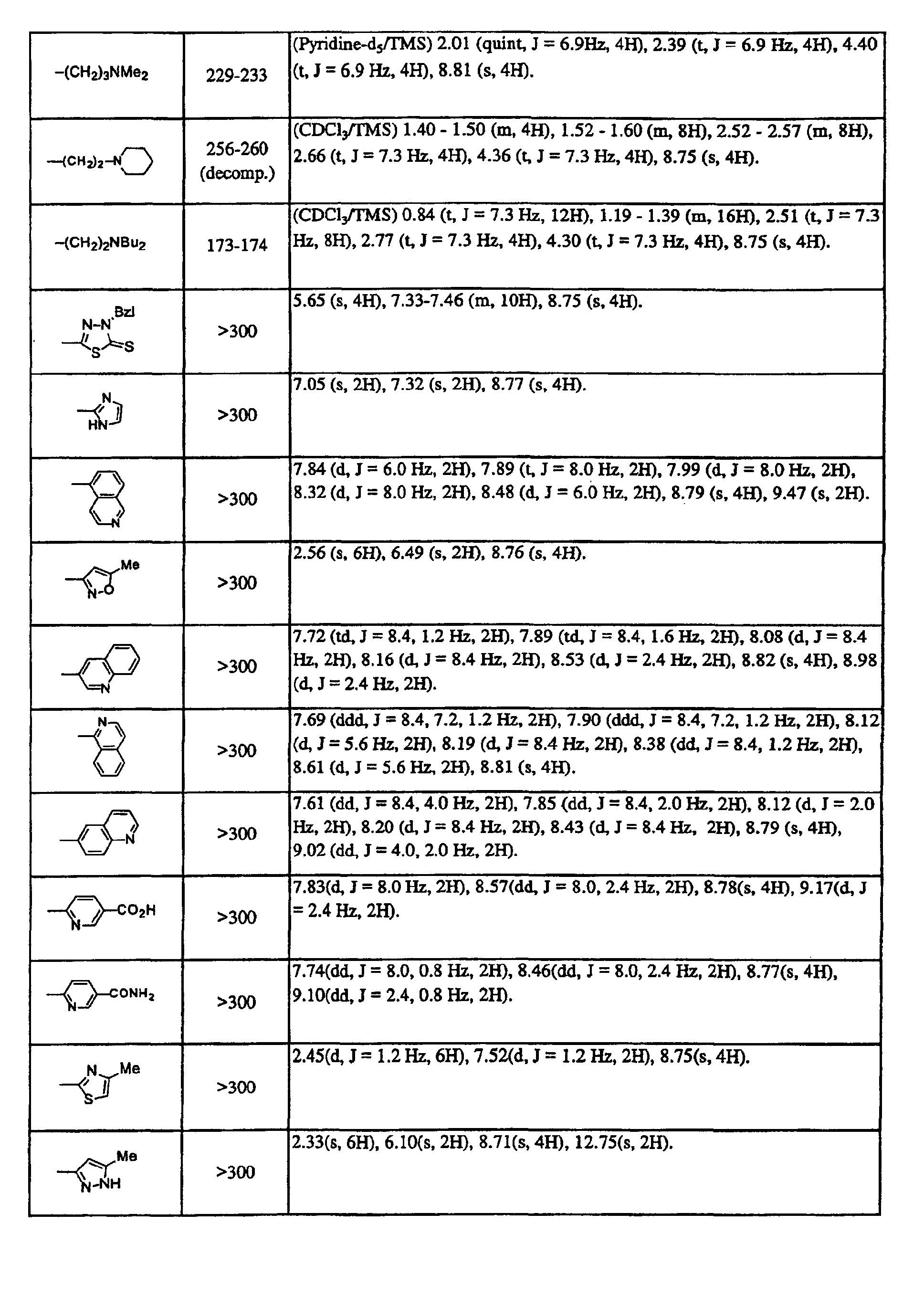

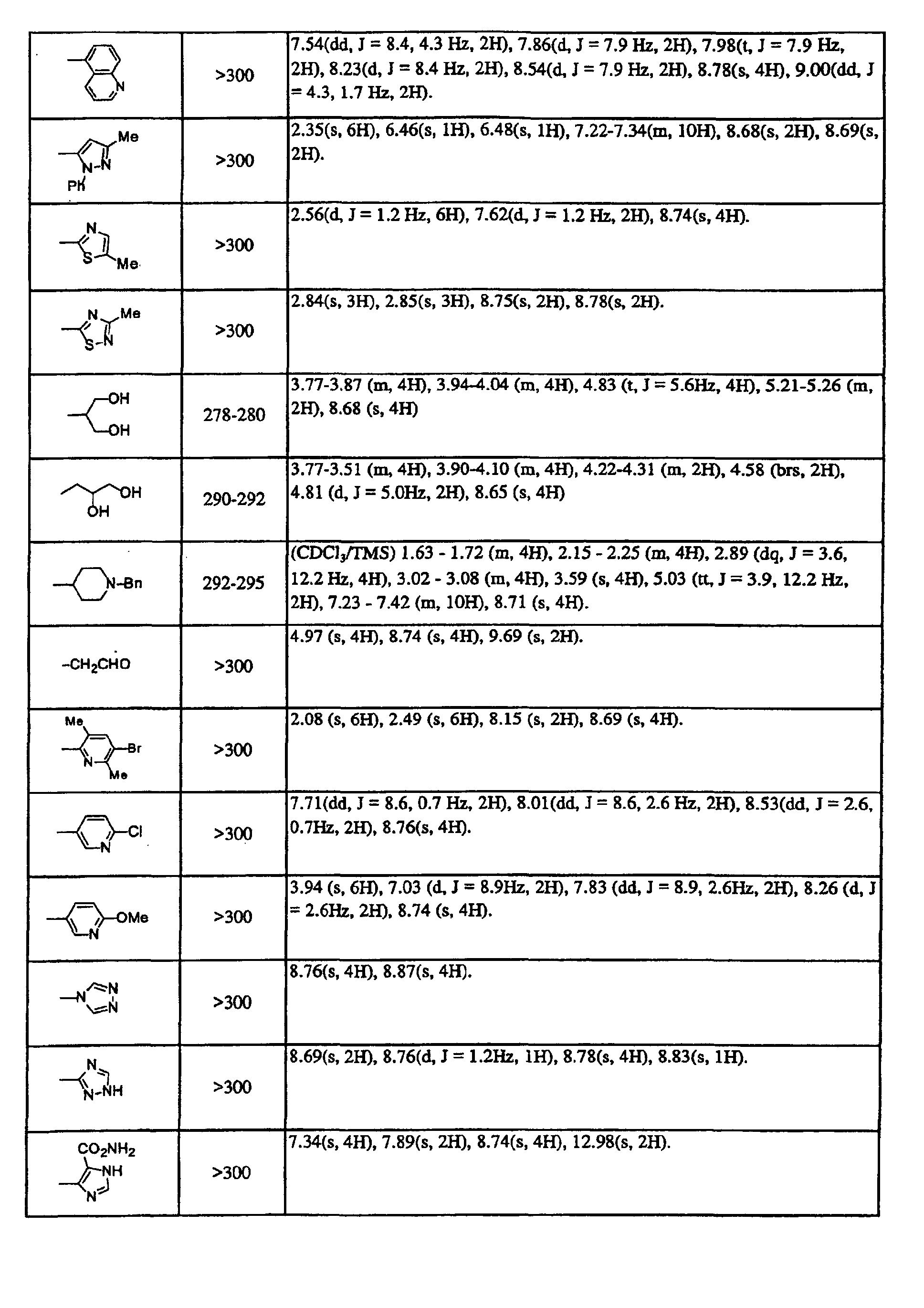

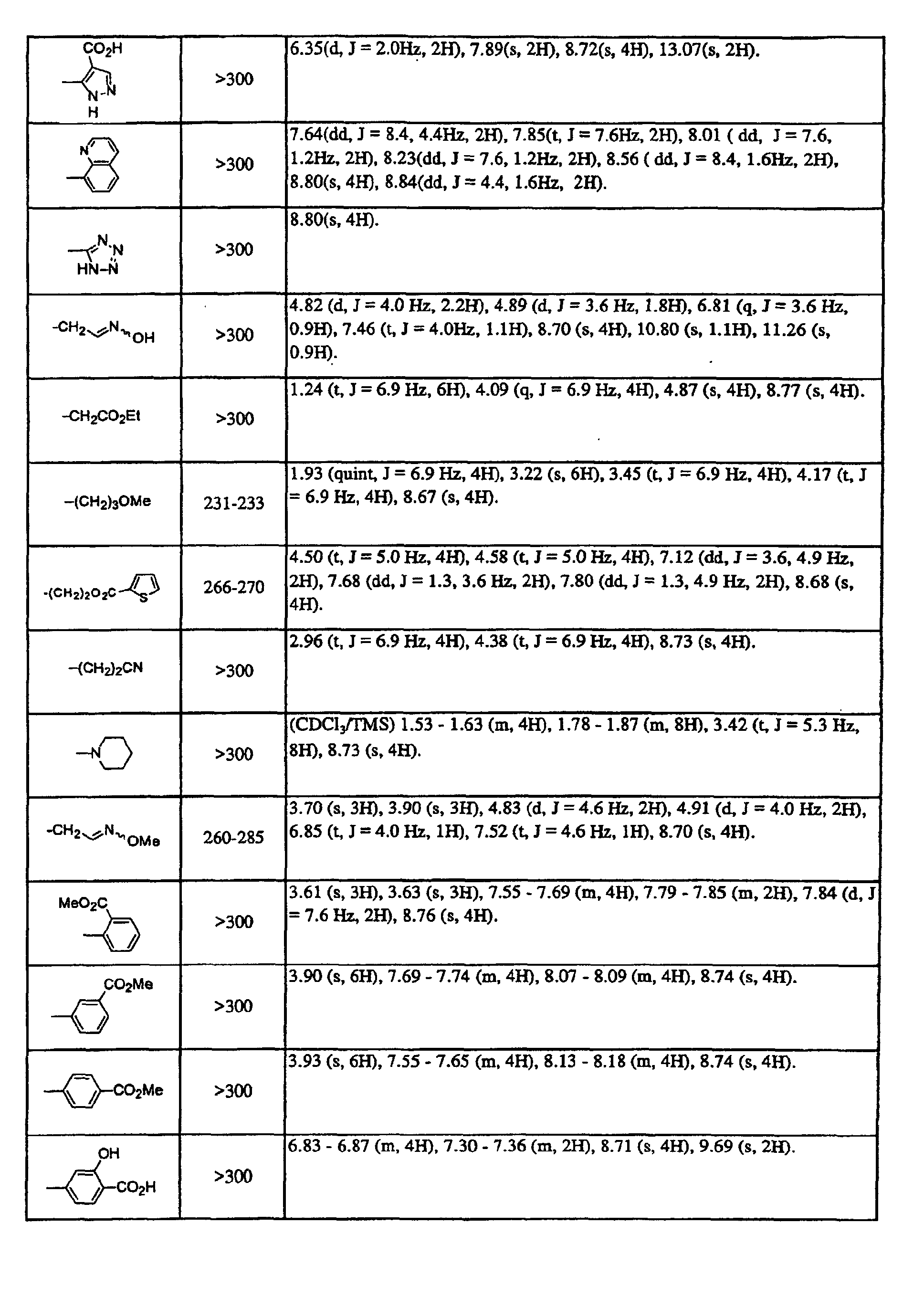

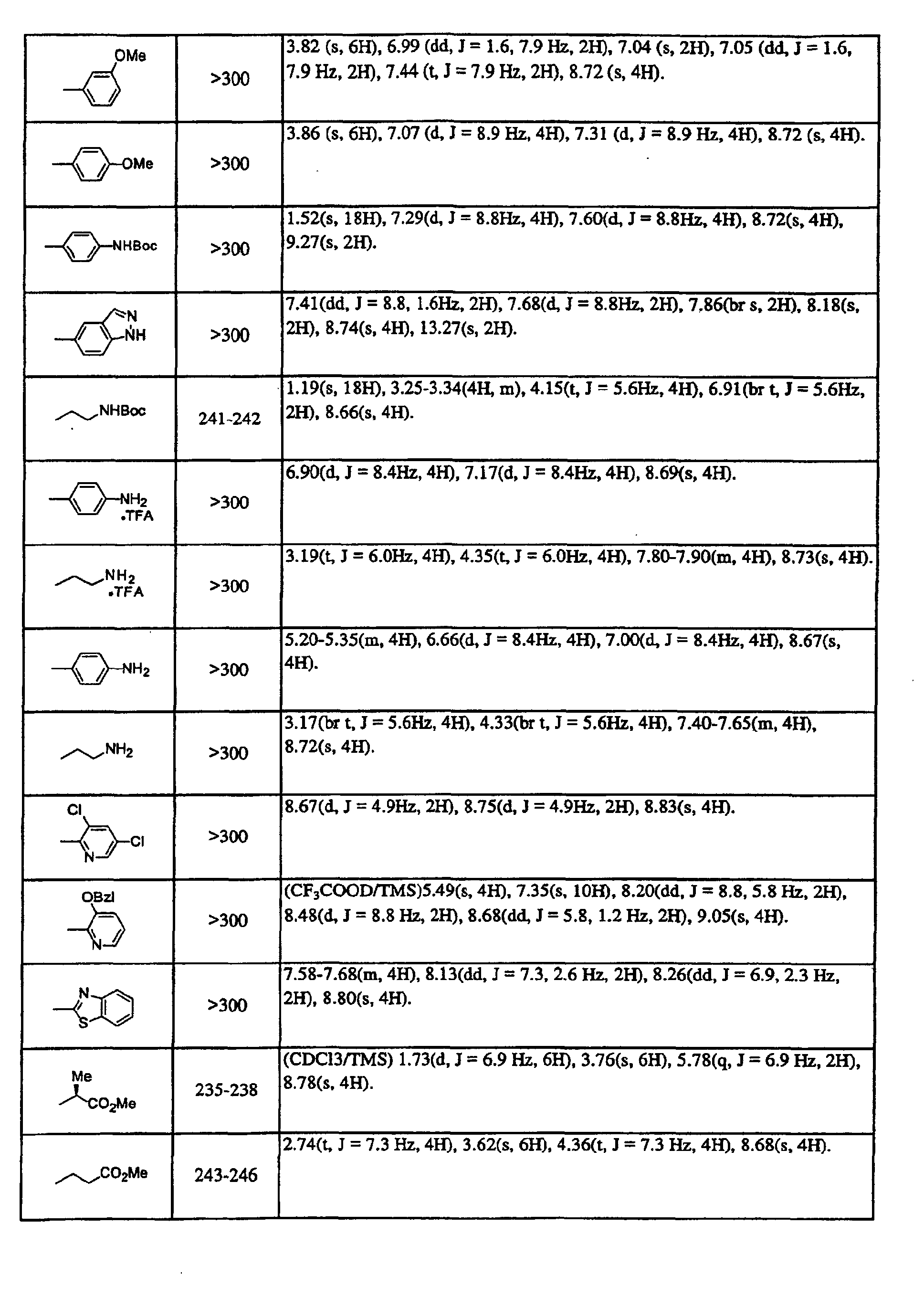

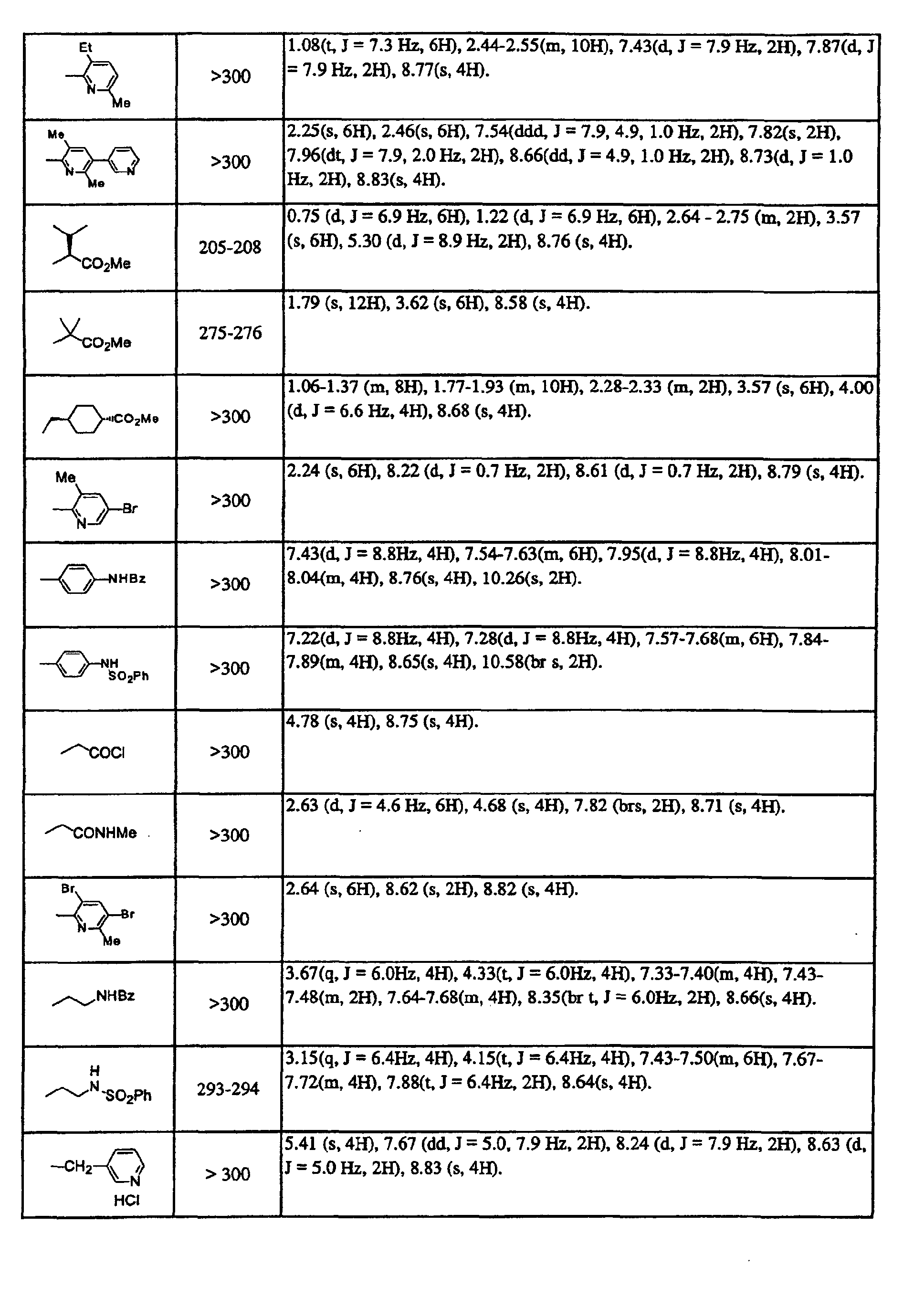

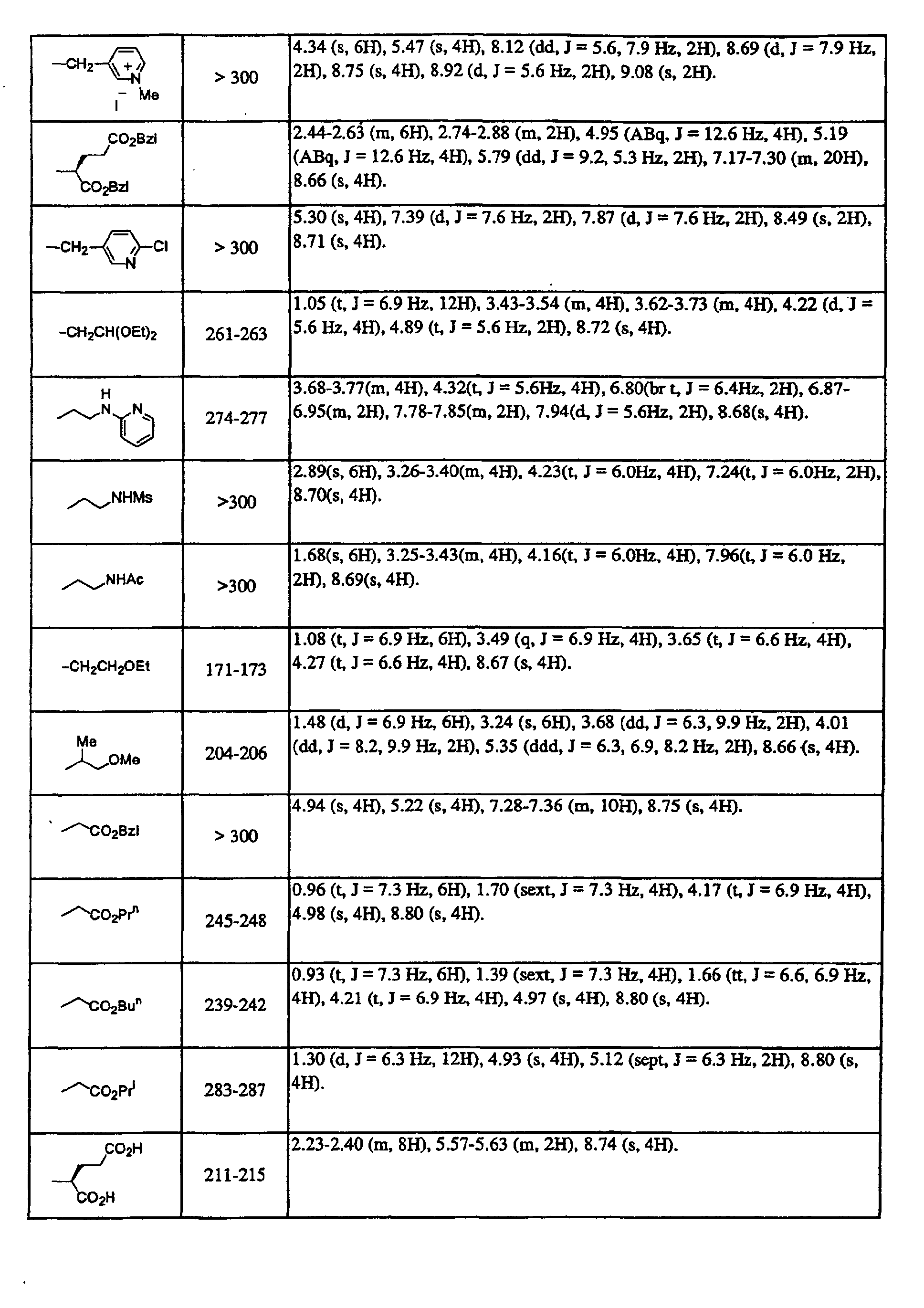

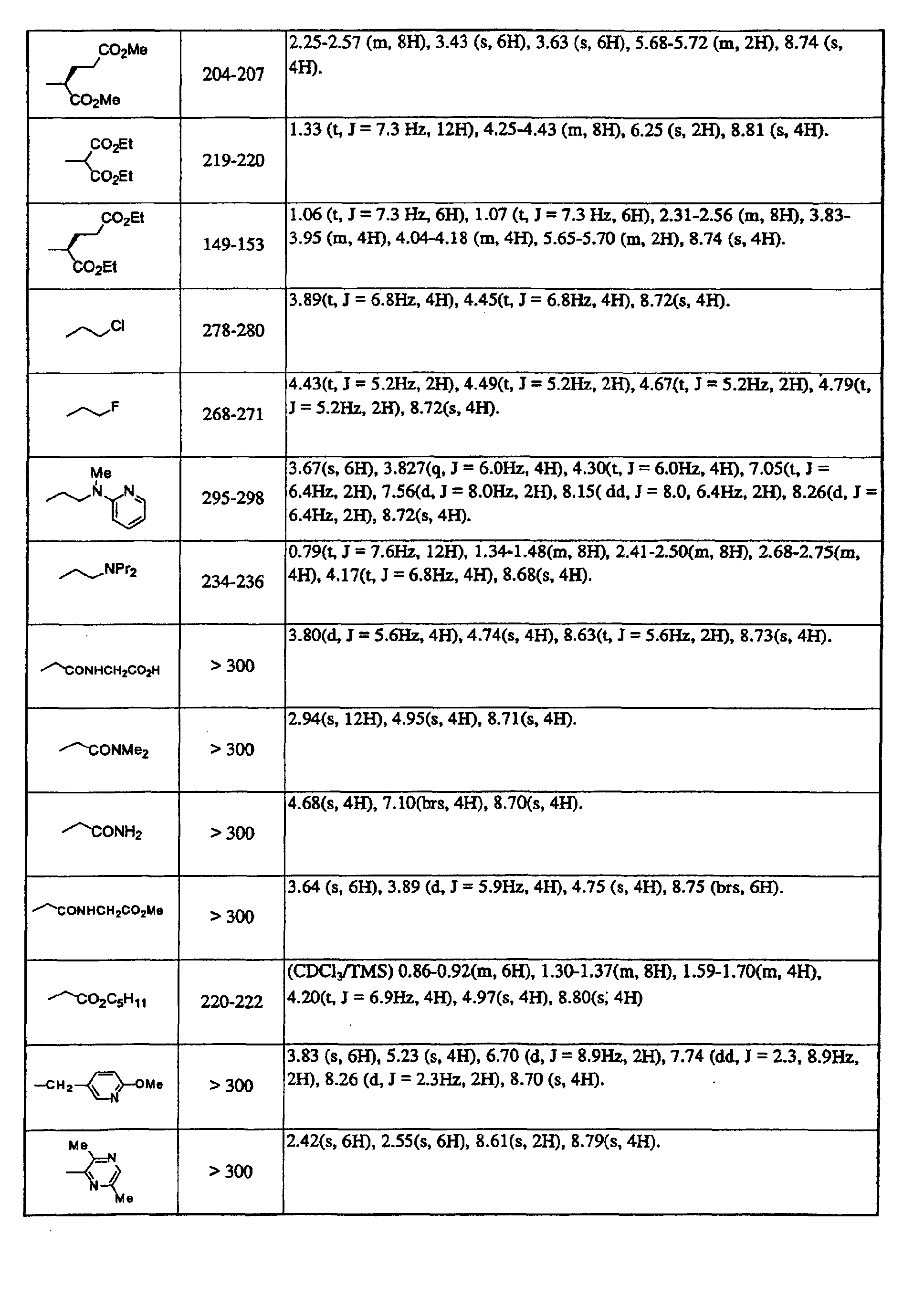

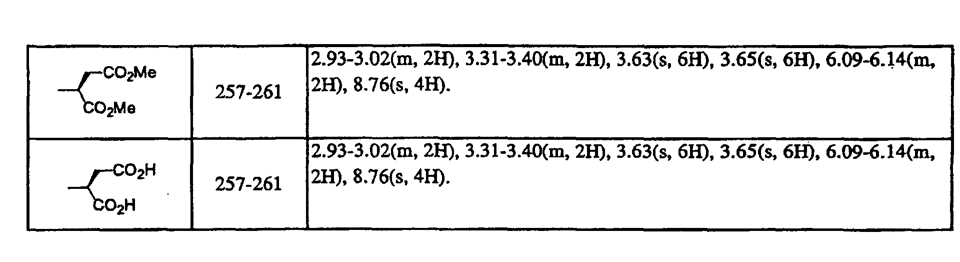

### (Example 5: in vitro testing of additional compounds)

Some of the compounds produced in Example 4 in which R¹ and R² are simultaneously any of substituents below were subjected to an in vitro test. 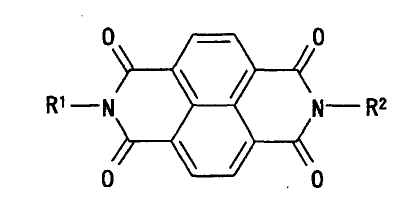 (where R¹ and R² are simultaneously any of the following substitutents 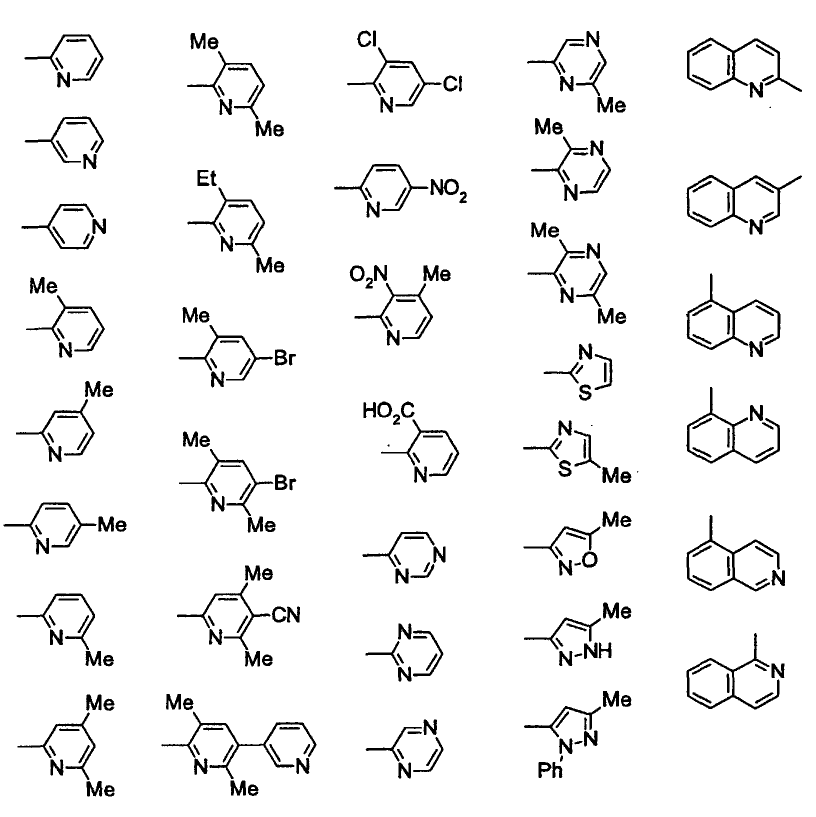 or any of the following substituents

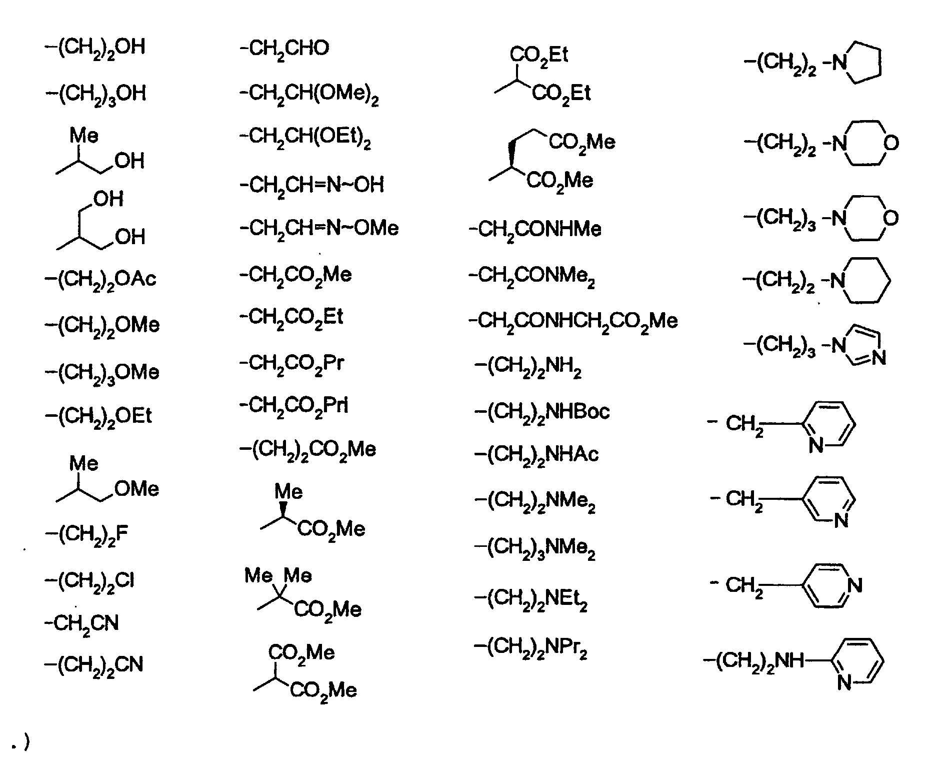

The antibacterial activity of a test compound was studied with a minute amount liquid dilution method using a 96-well microplate. Briefly, the compound of the present invention was dissolved in dimethylsulfoxide to prepare a solution having a concentration of 1 mg/ml. 100 µl of the 1 mg/ml solution of the test compound was poured into a first well containing 100 µl of growth medium which had been distributed over the microplate (Columbia liquid medium containing 0.1% β-cyclodextrin and 5% bovine fetal serum), followed by mixing. Subsequently, the mixture was serially diluted by two fold in a second well and thereafter to diluted mixtures of the test compound. 100 µl of H. pylori (ATCC 43629) bacterium solution which had been prepared to 10⁶ CFU/ml was added to the diluted mixtures, followed by incubation at 37°C for 48 hours in a 10% CO₂ incubator.

The antibacterial activity (MIC) against subject bacteria was judged by measuring the absorbance at a wavelength of 660 nm. Specifically, the absorbance of the antibacterial activity testing medium was used as a standard, and the maximum dilution ratio of the test compound that had an absorbance which was not beyond the absorbance of the antibacterial activity testing medium was defined as the antibacterial activity (MIC).

The results of the measurement of in vitro activity are shown in units of MIC (µg/ml) in a table below.

As is apparent from the table, R¹ and R² are heterocyclic group, substituted heterocyclic group, alkyl, or substituted alkyl; X¹, X², Y¹ and Y² are all hydrogen. Particularly, when R¹ and R² were separately pyridyl, substituted pyridyl, pyrimidyl, substituted pyrimidyl, pyrazyl, substituted pyrazyl, quinolyl, substituted quinolyl, isoquinolyl, and substituted isoquinolyl, or were separately substituted alkyl where the substituent of the substituted alkyl was pyridyl, hydroxy, substituted carboxy, alkoxy, or substituted amino, a preferable Helicobacter destorying activity was demonstrated. This compound can be used as a safe medicament without toxicity.

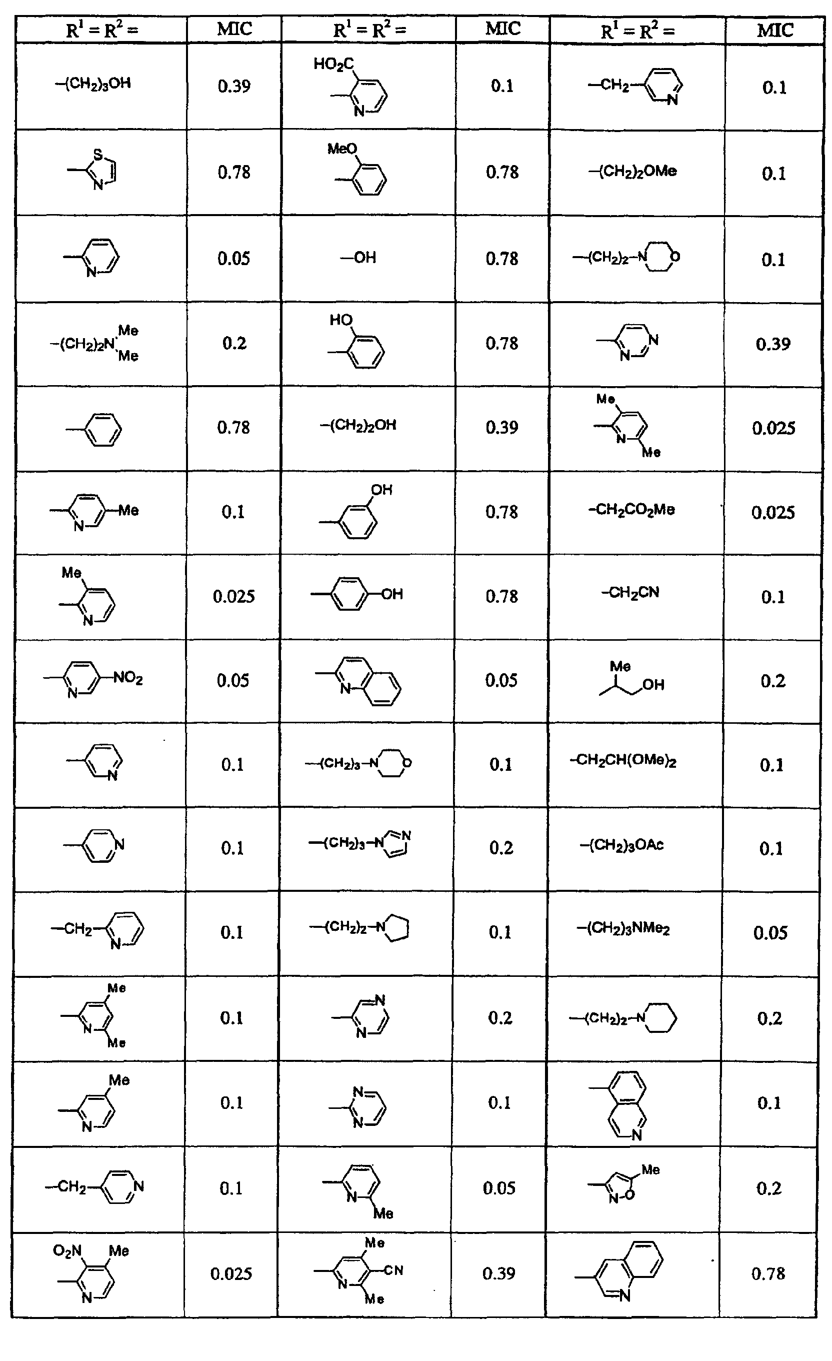

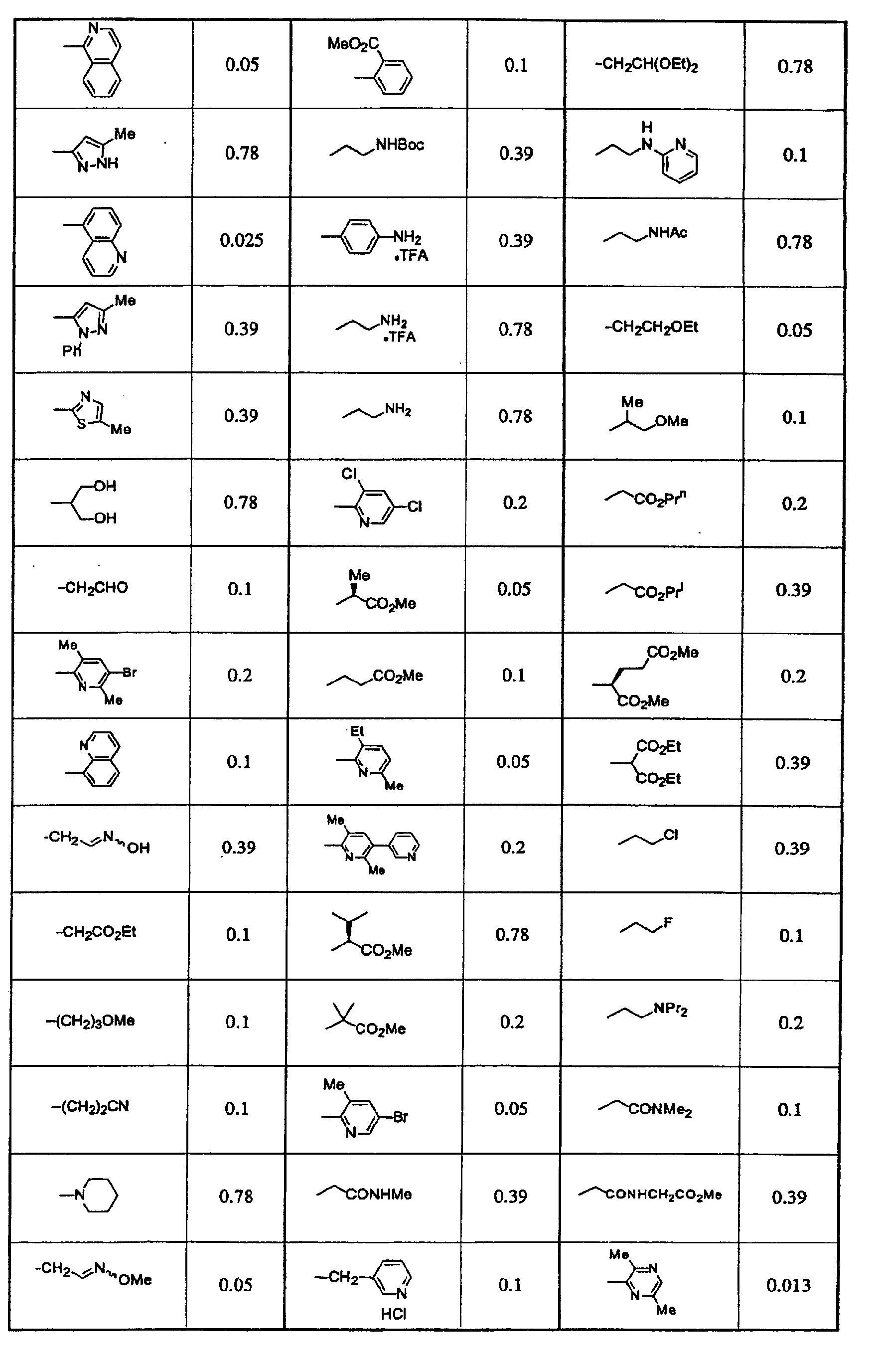

### INDUSTRIAL APPLICABILITY

According to the present invention, an anti-Helicobacter compound having a novel structure is provided. The present invention provides an anti-Helicobacter agent containing the compound of the present invention as a single component. The use of the compound of the present invention as a single component can reduce the side effects in treatment of a disease, such as peptic ulcers, which are otherwise caused by the use of a mixture of drugs, for example. The compound or composition of the present invention can specifically kill and eradicate Helicobacter, thereby making it possible to effectively treating peptic diseases (including, gastric ulcers, duodenal ulcers, and gastritis, for example).

## Claims

1. A compound represented by the following formula: 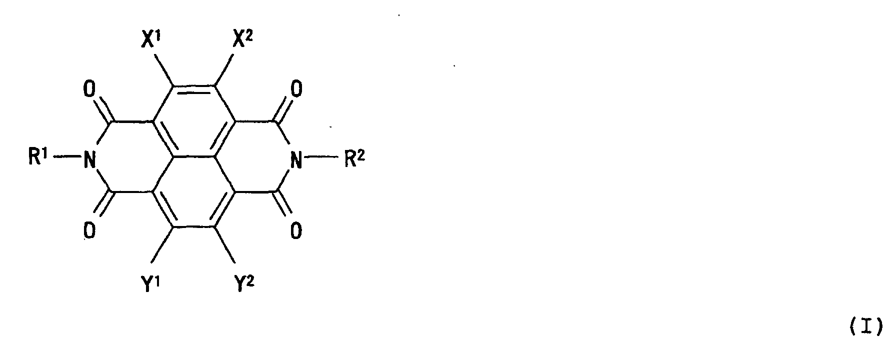 (where R¹ and R² are separately selected from the group consisting of hydrogen, alkyl, substituted alkyl, cycloalkyl, substituted cycloalkyl, alkenyl, substituted alkenyl, cycloalkenyl, substituted cycloalkenyl, alkynyl, substituted alkynyl, alkoxy, substituted alkoxy, carbocyclic group, substituted carbocyclic group, heterocyclic group, substituted heterocyclic group, halogen, hydroxy, substituted hydroxy, thiol, substituted thiol, cyano, nitro, amino, substituted amino, carboxy, substituted carboxy, acyl, substituted acyl, thiocarboxy, substituted thiocarboxy, amide, substituted amide, substituted carbonyl, substituted thiocarbonyl,substituted sulfonyl, and substituted sulfinyl; and X¹, X², Y¹, and Y² are separately selected from the group consisting of hydrogen, halogen, alkoxy, substituted alkoxy, amino, substituted amino, alkylthio, substituted alkylthio, arylthio, substituted arylthio, nitro, carboxy, substituted carboxy, acyl, substituted acyl, and substituted sulfonyl), or a pharmaceutically acceptable salt or hydrate thereof.

2. A compound or a pharmaceutically acceptable salt or hydrate thereof according to claim 1, wherein R¹ and R² are separately heterocyclic group, substituted heterocyclic group, alkyl, or substituted alkyl.

3. A compound or a pharmaceutically acceptable salt or hydrate thereof according to claim 1, wherein R¹ and R² are separately heterocyclic group, substituted heterocyclic group, alkyl, or substituted alkyl; and X¹, X², Y¹ and Y² are separately substituents selected from the group consisting of alkyl, halogen, and hydrogen.

4. A compound or a pharmaceutically acceptable salt or hydrate thereof according to claim 1, wherein R¹ and R² are separately heterocyclic group, substituted heterocyclic group, alkyl, or substituted alkyl; and X¹, X², Y¹ and Y² are all hydrogen.

5. A compound or a pharmaceutically acceptable salt or hydrate thereof according to claim 1, wherein R¹ and R² are separately selected from the group consisting of pyridyl, substituted pyridyl, pyrimidyl, substituted pyrimidyl, pyrazyl, substituted pyrazyl, quinolyl, substituted quinolyl, isoquinolyl, and substituted isoquinolyl.

6. A compound or a pharmaceutically acceptable salt or hydrate thereof according to claim 1, wherein R¹ and R² are separately substituted alkyl; and the substituent of the substituted alkyl is pyridyl, hydroxy, substituted carboxy, alkoxy, or substituted amino.

7. A pharmaceutical composition, comprising:
a compound represented by the following formula: 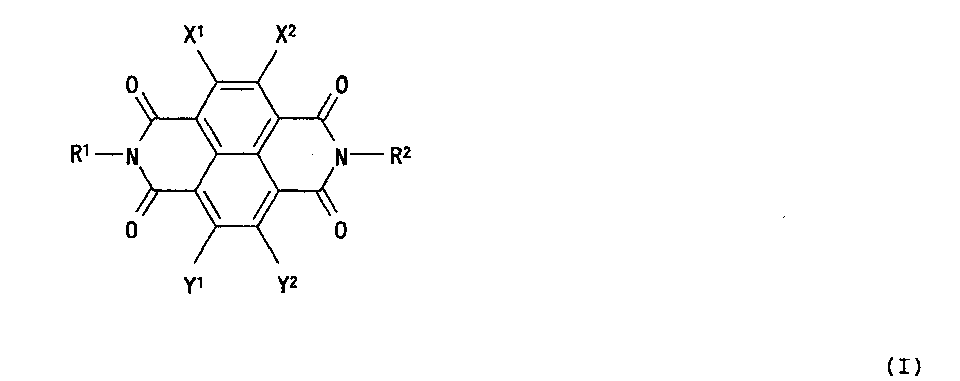 (where R¹ and R² are separately selected from the group consisting of hydrogen, alkyl, substituted alkyl, cycloalkyl, substituted cycloalkyl, alkenyl, substituted alkenyl, cycloalkenyl, substituted cycloalkenyl, alkynyl, substituted alkynyl, alkoxy, substituted alkoxy, carbocyclic group, substituted carbocyclic group, heterocyclic group, substituted heterocyclic group, halogen, hydroxy, substituted hydroxy, thiol, substituted thiol, cyano, nitro, amino, substituted amino, carboxy, substituted carboxy, acyl, substituted acyl, thiocarboxy, substituted thiocarboxy, amide, substituted amide, substituted carbonyl, substituted thiocarbonyl, substituted sulfonyl, and substituted sulfinyl; and
X¹, X², Y¹ and Y² are separately selected from the group consisting of hydrogen, halogen, alkoxy, substituted alkoxy, amino, substituted amino, alkylthio, substituted alkylthio, arylthio, substitutedarylthio, nitro, carboxy, substituted carboxy, acyl, substituted acyl, and substituted sulfonyl), or a pharmaceutically acceptable salt or hydrate thereof; and
a pharmaceutically acceptable carrier.

8. A pharmaceutical composition according to claim 7, wherein R¹ and R² are separately heterocyclic group, substituted heterocyclic group, alkyl, or substituted alkyl.

9. A pharmaceutical composition according to claim 7, wherein R¹ and R² are separately heterocyclic group, substituted heterocyclic group, alkyl, or substituted alkyl; and X¹, X², Y¹ and Y² are separately substituents selected from the group consisting of alkyl, halogen, and hydrogen.

10. A pharmaceutical composition according to claim 7, wherein R¹ and R² are separately heterocyclic group, substituted heterocyclic group, alkyl, or substituted alkyl; and X¹, X², Y¹ and Y² are all hydrogen.

11. A pharmaceutical composition according to claim 7, wherein R¹ and R² are separately selected from the group consisting of pyridyl, substituted pyridyl, pyrimidyl, substituted pyrimidyl, pyrazyl, substituted pyrazyl, quinolyl, substituted quinolyl, isoquinolyl, and substituted isoquinolyl.

12. A pharmaceutical composition according to claim 7, wherein R¹ and R² are separately substituted alkyl; and the substituent of the substituted alkyl is pyridyl, hydroxy, substituted carboxy, alkoxy, or substituted amino.

13. A pharmaceutical composition according to claim 7, wherein the composition is an anti-Helicobacter pharmaceutical composition.

14. A pharmaceutical composition according to claim 7, further containing at least one drug selected from the group of consisting of an antibacterial agent, a mucosal protection agent promoter, an anti-gastrin agent, a H₂ receptor antagonist, a proton pump inhibitor, a bismuth preparation, and a gastrointestinal drug.

15. A pharmaceutical composition according to claim 7, wherein a disease to be treated by the composition is gastric ulcer, duodenal ulcer, or gastritis.

16. An anti-Helicobacter pharmaceutical composition according to claim 13, wherein the Helicobacter is Helicobacter pylori or Helicobacter felis.

17. A pharmaceutical composition according to claim 7,
wherein the composition is used to enhance the efficacy of at least one drug selected from the group consisting of an antibacterial agent, a mucosal protection agent promoter, an anti-gastrin agent, a H₂ receptor antagonist, a proton pump inhibitor, abismuth preparation, and a gastrointestinal drug.

18. A method for treating or preventing a disease caused by Helicobacter, or preventing the recurrence of the disease, the method comprising the step of:
a) administering to a patient with a disease a formulation containing a compound represented by the following formula: 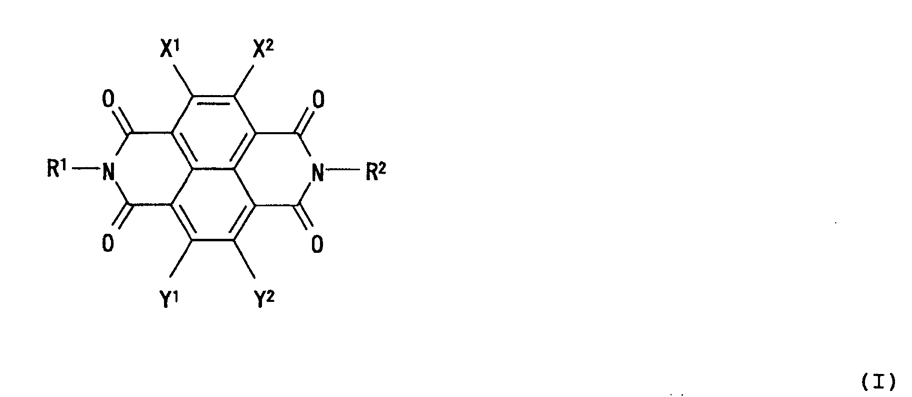 (where R¹ and R² are separately selected from the group consisting of hydrogen, alkyl, substituted alkyl, cycloalkyl, substituted cycloalkyl, alkenyl, substituted alkenyl, cycloalkenyl, substituted cycloalkenyl, alkynyl, substituted alkynyl, alkoxy, substituted alkoxy, carbocyclic group, substituted carbocyclic group, heterocyclic group, substituted heterocyclic group, halogen, hydroxy, substituted hydroxy, thiol, substituted thiol, cyano, nitro, amino, substituted amino, carboxy, substituted carboxy, acyl, substituted acyl, thiocarboxy, substituted thiocarboxy, amide, substituted amide, substituted carbonyl, substituted thiocarbonyl, substituted sulfonyl, and substituted sulfinyl; and
X¹, X², Y¹ and Y² are separately selected from the group consisting of hydrogen, halogen, alkoxy, substituted alkoxy, amino, substituted amino, alkylthio, substituted alkylthio, arylthio, substituted arylthio, nitro, carboxy, substituted carboxy, acyl, substituted acyl, and substituted sulfonyl), or a pharmaceutically acceptable salt or hydrate thereof.

19. A method according to claim 18, wherein R¹ and R² are separately heterocyclic group, substituted heterocyclic group, alkyl, or substituted alkyl.

20. A method according to claim 18, wherein R¹ and R² are separately heterocyclic group, substituted heterocyclic group, alkyl, or substituted alkyl; and X¹, X², Y¹ and Y² are separately substituents selected from the group consisting of alkyl, halogen, and hydrogen.

21. A method according to claim 18, wherein R¹ and R² are separately heterocyclic group, substituted heterocyclic group, alkyl, or substituted alkyl; and X¹, X², Y¹ and Y² are all hydrogen.

22. A method according to claim 18, wherein R¹ and R² are selected from the group consisting of pyridyl, substituted pyridyl, pyrimidyl, substituted pyrimidyl, pyrazyl, substituted pyrazyl, quinolyl, substituted quinolyl, isoquinolyl, and substituted isoquinolyl.

23. A method according to claim 18, wherein R¹ and R² are separately substituted alkyl; and the substituent of the substituted alkyl is pyridyl, hydroxy, substituted carboxy, alkoxy, or substituted amino.

24. A method according to claim 18, wherein the formulation contains a pharmaceutically acceptable carrier.

25. A method according to claim 18, wherein the formulation further containing at least one drug selected from the group of consisting of an antibacterial agent, a mucosal protection agent promoter, an anti-gastrin agent, a H₂ receptor antagonist, a proton pump inhibitor, a bismuth preparation, and a gastrointestinal drug.

26. A method according to claim 18, wherein the disease is gastric ulcer, duodenal ulcer, or gastritis.

27. A method according to claim 18, wherein the Helicobacter is Helicobacter pylori or Helicobacter felis.

28. Use of a compound according to any of claims 1-6 for use in treating or preventing a disease caused by Helicobacter, or preventing the recurrence of the disease.
